# EUROPEAN PATENT APPLICATION

(11) **EP 4 043 547 A1**
(43) Date of publication of application: **17.08.2022**
(21) Application number: 19947323.2
(22) Date of filing: 27.09.2019
(51) Int. Cl.: C12M 1/34

(54) **INFORMATION PROCESSING DEVICE, INFORMATION PROCESSING METHOD, INFORMATION PROCESSING PROGRAM, AND INFORMATION PROCESSING SYSTEM**

(71) Applicant: NIKON CORPORATION, Minato-ku Tokyo 108-6290 (JP)
(72) Inventor: ISHIKAWA, Momotaro, Tokyo 108-6290 (JP); TADAKI, Toshihide, Tokyo 108-6290 (JP); ABE, Takeyuki, Tokyo 108-6290 (JP); HAYASHI, Kohma, Tokyo 108-6290 (JP); NEGISHI, Kyoko, Tokyo 140-0015 (JP); SENDODA, Ryoko, Tokyo 140-0015 (JP)
(74) Representative: Hoffmann Eitle
(86) International application number: PCT/JP2019/038164
(87) International publication number: WO 2021/059488

(57) **Abstract**

An information processing device includes: an acquirer that acquires observation results obtained from a time-lapse image of a target object; and a controller that indicates, in a display image, analysis results visually representing the observation results and an increase/decrease time indicating an amount of time required to increase or decrease a predetermined value related to the target object. When a setting for a section in the analysis results has been received, the controller indicates, in the display image, the increase/decrease time corresponding to the section that has been set.

## Description

### Technical Field

The present invention relates to an information processing device, an information processing method, an information processing program, and an information processing system.

### Background Art

Patent Literature 1 discloses a technique for calculating, on the basis of an image of a colony of cells, a cell density from the area of the colony and the number of the cells contained in the colony of which the area has been calculated. For example, an analysis using such a technique yields a vast amount of results through day-today analytical work, and there is therefore a demand for a technique that enables a user to easily perform visual confirmation on those analytical results.

### Citation List

### Patent Literature

[Patent Literature 1] International Publication No. 2015/193951

### Summary of Invention

A first aspect of the invention is to provide an information processing device comprising: an acquirer that acquires observation results obtained from a time-lapse image of a target object; and a controller that indicates, in a display image, analysis results visually representing the observation results and an increase/decrease time indicating an amount of time required to increase or decrease a predetermined value related to the target object. wherein, when a setting for a section in the analysis results has been received, the controller indicates, in the display image, the increase/decrease time corresponding to the section that has been set.

A second aspect of the invention is to provide an information processing method comprising: acquiring observation results obtained from a time-lapse image of a target object; indicating, in a display image, analysis results visually representing the observation results and an increase/decrease time indicating an amount of time required to increase or decrease a predetermined value related to the target object; and when a setting for a section in the analysis results has been received, indicating, in the display image, the increase/decrease time corresponding to the section that has been set.

A third aspect of the invention is to provide an information processing program that causes a computer to execute processes of: acquiring observation results obtained from a time-lapse image of a target object; indicating, in a display image, analysis results visually representing the observation results and an increase/decrease time indicating an amount of time required to increase or decrease a predetermined value related to the target object; and when a setting for a section in the analysis results has been received, indicating, in the display image, the increase/decrease time corresponding to the section that has been set.

A fourth aspect of the invention is to provide an information processing system that outputs a display image to a user terminal by cloud computing, the information processing system comprising a server, wherein the server includes an acquirer that acquires observation results obtained from a time-lapse image of a target object, an image generator that generates the display image including analysis results visually representing the observation results and an increase/decrease time indicating an amount of time required to increase or decrease a predetermined value related to the target object, and an outputter that outputs the display image generated by the image generator to the user terminal via a network, and when a setting for a section in the analysis results has been received, the image generator indicates, in the display image, the increase/decrease time corresponding to the section that has been set.

The summary clause does not necessarily describe all necessary features of the embodiments of the present invention. The present invention may also be a subcombination of the features described above.

### Brief Description of the Drawings

Fig. 1 is a diagram showing an overall configuration example of an analysis system including an information processing device according to a first embodiment.
Fig. 2 is a diagram showing a configuration example of a culturing system connected to the information processing device according to the first embodiment.
Fig. 3 is a block diagram showing a configuration example of the culturing system connected to the information processing device according to the first embodiment.
Fig. 4 is a diagram for describing an example of connections around a control unit of the culturing system connected to the information processing device according to the first embodiment.
Fig. 5 is a block diagram showing a functional configuration example of the information processing device according to the first embodiment.
Fig. 6 is a diagram showing an example of information stored in a memory storage device according to the first embodiment.
Fig. 7 is a diagram showing a screen example of group registration according to the first embodiment.
Fig. 8 is a diagram showing a screen example of the group registration according to the first embodiment.
Fig. 9 is a diagram showing a screen example of the group registration according to the first embodiment.
Fig. 10 is a diagram showing a screen example of the group registration according to the first embodiment.
Fig. 11 is a diagram showing a screen example of the group registration according to the first embodiment.
Fig. 12 is a diagram showing a screen example of the group registration according to the first embodiment.
Fig. 13 is a diagram showing a screen example of the group registration according to the first embodiment.
Fig. 14 is a diagram showing a screen example of the group registration according to the first embodiment.
Fig. 15 is a diagram showing a screen example of the group registration according to the first embodiment.
Fig. 16 is a diagram showing a screen example of the group registration according to the first embodiment.
Fig. 17 is a flowchart showing an example of a flow of a process in the information processing device according to the first embodiment.
Fig. 18 is a flowchart showing an example of a flow of a process for switching observation image display according to the first embodiment.
Fig. 19 is a block diagram showing a functional configuration example of an information processing device according to a second embodiment.
Fig. 20 is a diagram showing an example of a display image including an analysis result and a doubling time according to the second embodiment.
Fig. 21 is a diagram showing an example of a display image including an analysis result and a doubling time according to the second embodiment.
Fig. 22 is a diagram showing an example of a display image including an analysis result and a doubling time according to the second embodiment.
Fig. 23 is a diagram showing an example of a display image including an analysis result and a doubling time according to the second embodiment.
Fig. 24 is a flowchart showing an example of a flow of a process in the information processing device according to the second embodiment.
Fig. 25 is a flowchart showing an example of a flow of a process of displaying a doubling time corresponding to a setting of a section according to the second embodiment.
Fig. 26 is a block diagram showing a functional configuration example of an information processing device according to a third embodiment.
Fig. 27 is a diagram for describing an example of normalization of analysis results according to the third embodiment.
Fig. 28 is a diagram for describing an example of an operation for normalizing analysis results according to the third embodiment.
Fig. 29 is a diagram for describing an example of an operation for denormalizing analysis results according to the third embodiment.
Fig. 30 is a flowchart showing an example of a flow of a process in the information processing device according to the third embodiment.
Fig. 31 is a diagram showing a configuration example of an information processing system according to a fourth embodiment.

### Description of Embodiments

Hereunder, embodiments of the present invention will be described, with reference to the drawings. It should be noted that, in the drawings, scale is changed as necessary to illustrate the embodiments, such as by enlarging or by emphasizing a part, and thus, the embodiments may differ from the actual product in size and shape in some cases.

### First Embodiment

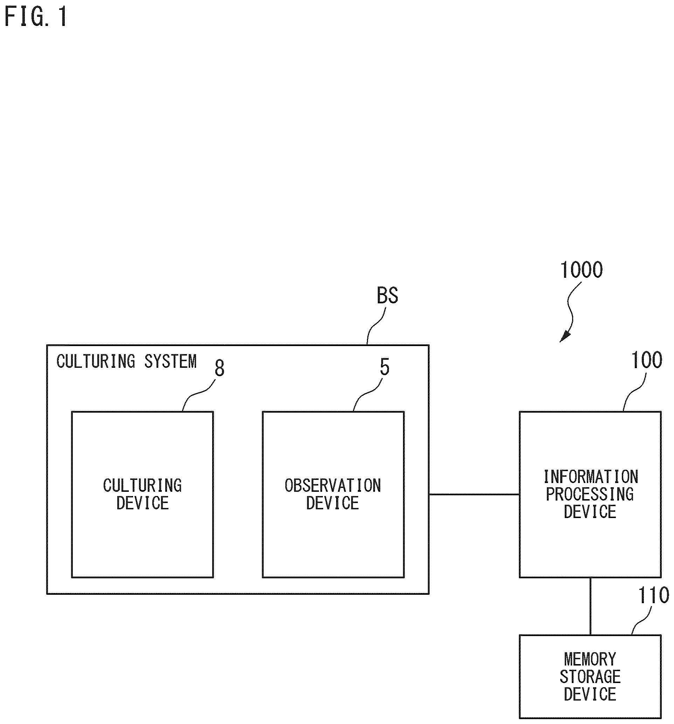

Hereunder, a first embodiment will be described. Fig. 1 is a diagram showing an overall configuration example of an analysis system including an information processing device according to the first embodiment. As shown in Fig. 1, an analysis system 1000 includes a culturing system BS, an information processing device 100, and a memory storage device 110. The culturing system BS includes a culturing device 8 and an observation device 5. The analysis system 1000 is a system for culturing target objects (for example, cells, samples, or specimens), observing (image-capturing) the process of culturing, and analyzing observation results (for example, captured images).

The culturing system BS, the information processing device 100, and the memory storage device 110 are connected via a network such as the Internet, LAN (Local Area Network), or WAN (Wide Area Network). The culturing system BS, the information processing device 100, and the memory storage device 110 may also be connected via a network composed of a combination of the Internet, a LAN (Local Area Network), and a WAN (Wide Area Network). Such a network is not limited to a wired communication network, and may include a wireless communication network. The information processing device 100 may include the memory storage device 110. The culturing system BS and the memory storage device 110 may be connected via a network.

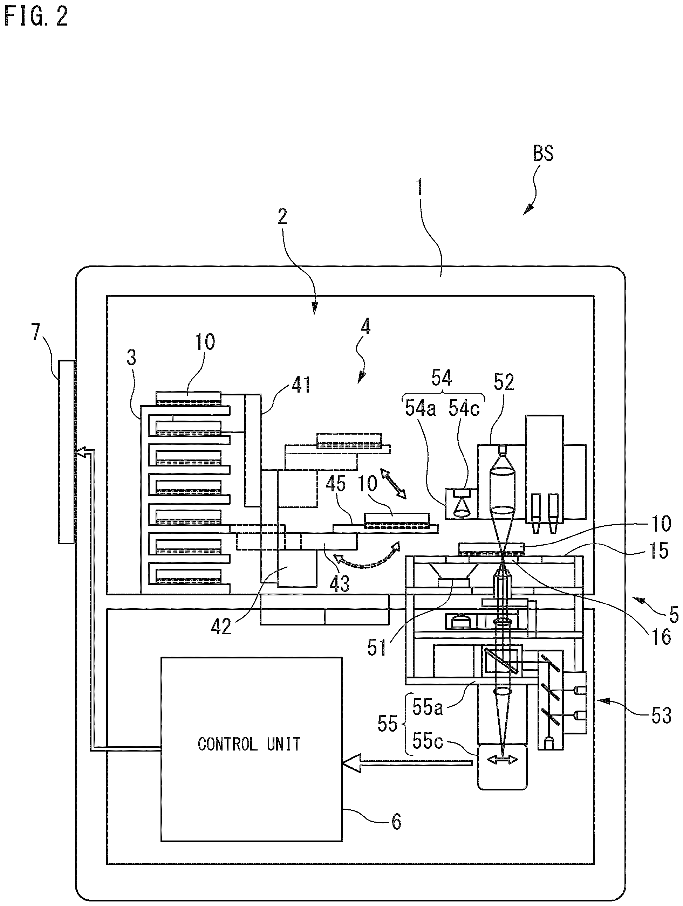

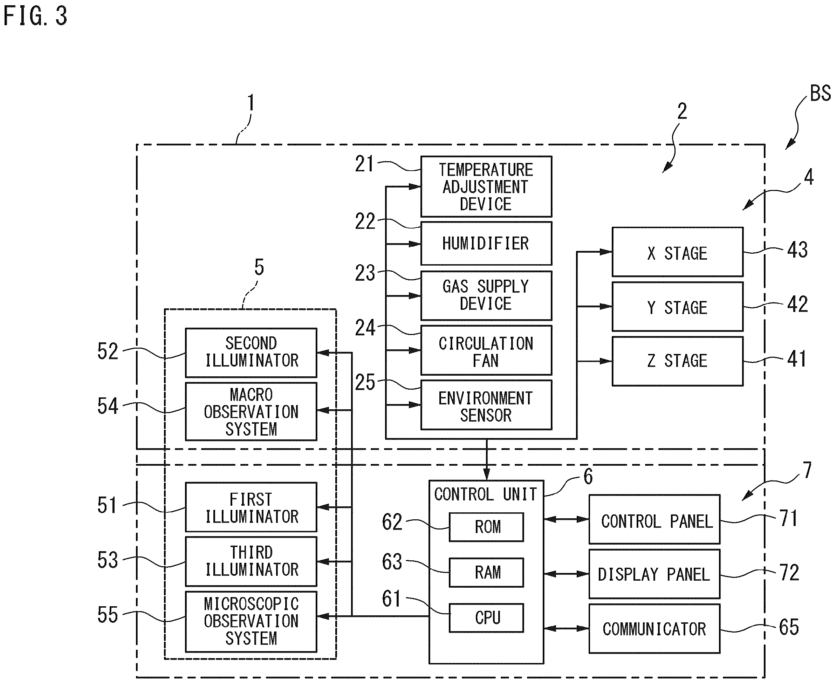

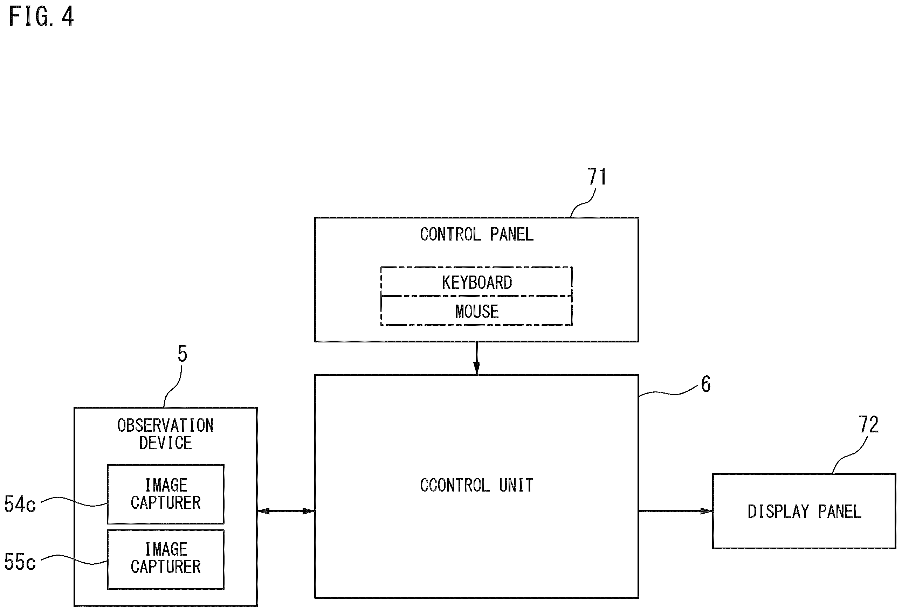

Fig. 2 is a diagram showing a configuration example of the culturing system connected to the information processing device according to the first embodiment. Fig. 3 is a block diagram showing a configuration example of the culturing system connected to the information processing device according to the first embodiment. Fig. 4 is a diagram for describing an example of connections around a control unit of the culturing system connected to the information processing device according to the first embodiment.

Broadly speaking, the culturing system BS has a culturing chamber 2 provided in an upper part of a casing 1, a stocker 3 that stores and holds a plurality of culture vessels 10, an observation device 5 that observes (image-captures) target objects in the culture vessels 10, and a transport unit (transport device) 4 that transports the culture vessels 10. In addition, the culturing system BS has a control unit (control device) 6 that controls the operation of the system, and an operation panel 7 including a display device. The culturing chamber 2, the stocker 3, the transport unit 4, and so forth correspond to the culturing device 8.

The culturing chamber 2 is a chamber that forms a culturing environment in microscopic observation for observation objects such as cells. In the culturing chamber 2 there are provided a temperature adjustment device 21, a humidifier 22, a gas supply device 23, a circulation fan 24, and an environment sensor 25. The temperature adjustment device 21, in coordination with the environment sensor 25, adjusts the temperature within the culturing chamber 2 to a predetermined set temperature. The humidifier 22, in coordination with the environment sensor 25, adjusts the humidity within the culturing chamber 2 to a predetermined set humidity. The gas supply device 23 supplies CO₂ gas, N₂ gas, O₂ gas, or the like, in coordination with the environment sensor 25. The circulation fan 24 is a fan that, in coordination with the environment sensor 25, circulates the gas (air) within the culturing chamber 2 to thereby adjust the temperature. The environment sensor 25 detects the temperature, the humidity, the carbon dioxide concentration, nitrogen concentration, oxygen concentration, or the like within the culturing chamber 2.

The stocker 3 is formed in the shape of a rack that is sectioned along the front-rear direction as well as along the upper-lower direction. Each rack shelf has, for example, a unique address set therefor. Culture vessels 10 are appropriately selected according to the type and purpose of the target object to be cultured. The culture vessels 10 may be, for example, well plates, flasks, or dish type culture vessels. In the present embodiment, well plates are used as an example. Target objects are injected into the culture vessel 10 together with a liquid medium (culture fluid) and held thereon. For example, a code number is assigned to each of the culture vessels 10. Each culture vessel 10 is stored in the stocker 3 in association with the specified address thereof, according to the code number assigned thereto. The transport unit 4 has a Z stage 41 that can move up and down, a Y stage 42 that can move forward and backward, and an X stage 43 that can move left and right, which are provided inside the culturing chamber 2. A supporting arm 45 lifts and supports the culture vessel 10 on the distal end side of the X stage 43.

The observation device 5 has a first illuminator 51, a second illuminator 52, a third illuminator 53, a macro observation system 54, a microscopic observation system 55, and a control unit 6. The first illuminator 51 illuminates a target object from below a sample stage 15. The second illuminator 52 illuminates a target object from above the sample stage 15, along the optical axis of the microscopic observation system 55. The third illuminator 53 illuminates a target object from below the sample stage 15, along the optical axis of the microscopic observation system 55. The macro observation system 54 performs a macro observation of a target object. The microscopic observation system 55 performs a micro observation of a target object. In the sample stage 15, a transparent window 16 composed of a material such as glass is provided within an observation region of the microscopic observation system 55.

The macro observation system 54 has an observation optical system 54a and an image capturer 54c such as a CCD camera that captures an image of a target object formed by the observation optical system 54a. The macro observation system 54 acquires an overall observation image from above the culture vessel 10 backlit by the first illuminator 51. The microscopic observation system 55 has an observation optical system 55a including an objective lens, an intermediate variable magnification lens, and a fluorescence filter, and an image capturer 55c such as a cooled CCD camera that captures an image of a target object formed by the observation optical system 55a. A plurality of the objective lenses and a plurality of the intermediate variable magnification lenses may respectively be provided. The objective lens and the intermediate variable magnification lens can be set for any observation magnification by changing the combination of lenses. The microscopic observation system 55 acquires a transmitted image of a target object illuminated by the second illuminator 52, a reflected image of the target object illuminated by the third illuminator 53, and a fluorescence image of the target object illuminated by the third illuminator 53. That is to say, the microscopic observation system 55 acquires a microscopic observation image obtained by microscopically observing a target object in the culture vessel 10.

The control unit 6 processes signals input from the image capturer 54c of the macro observation system 54 and the image capture 55c of the microscopic observation system 55, and generates images such as an overall observation image and a microscopic observation image. The control unit 6 performs image analysis on overall observation images and microscopic observation images to generate a time-lapse image. The control unit 6 outputs the generated image to the information processing device 100 and stores it in the memory storage device 110.

The control unit 6 has a CPU (Central Processing Unit) (processor) 61, a ROM (Read-Only Memory) 62, and a RAM (Random Access Memory) 63. The CPU 61 performs overall control of the control unit 6 and executes various processes in the control unit 6. The ROM 62 stores a control program and control data related to the culturing system BS. The RAM 63 includes an auxiliary memory storage device such as a hard disk or a DVD (Digital Versatile Disc), and temporarily stores observation conditions, image data, and so forth. Each of the units such as the culturing chamber 2, the transport unit 4, the observation device 5, and the operation panel 7 is connected to the control unit 6 (see Fig. 3).

The RAM 63 stores, for example, environmental conditions of the culturing chamber 2 according to an observation program, an observation schedule, an observation type, an observation position, an observation magnification, and so forth in the observation device 5. The RAM 63 includes a memory region for storing image data captured by the observation device 5, and stores the image data in association with index data including the code number of the culture vessel 10, the date and time of image capturing, and so forth. The operation panel 7 has an operational panel (operator, inputter) 71 and a display panel 72. The operational panel 71 includes input/output devices (operator, inputter) such as a keyboard, a mouse, and a switch. The user operates the operational panel 71 to input observation program settings, condition selections, operation instructions, and so forth. The communicator 65 is compliant with wired and wireless communication standards, and transmits and receives data to and from the observation device 5, the culturing system BS, or external devices (for example, a server, user's client terminal) connected to the control unit 6. Various types of information stored in the RAM 63 can be appropriately stored in the memory storage device 110 via the information processing device 100.

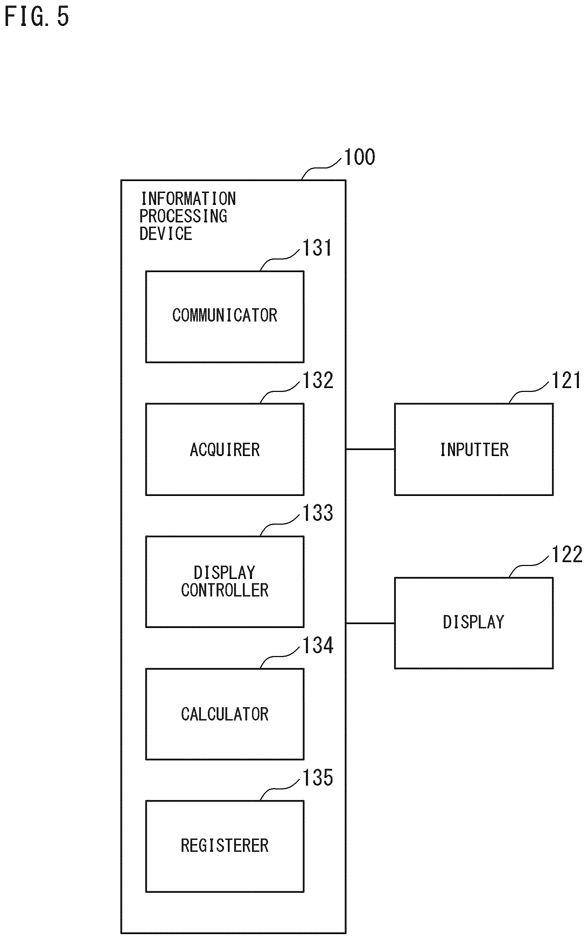

Fig. 5 is a block diagram showing a functional configuration example of the information processing device according to the first embodiment. As shown in Fig. 5, the information processing device 100 has a communicator 131, an acquirer 132, a display controller 133, a calculator 134, and a registerer 135. An inputter 121 and a display 122 are connected to the information processing device 100.

The inputter 121 accepts various operations performed by the user of the information processing device 100 and outputs control signals according to the user operation. The inputter 121 includes, for example, a mouse and a keyboard. The display 122 outputs display of various information (information including images) according to the user operation performed on the inputter 121. The display 122 includes, for example, a display or the like. The inputter 121 and the display 122 may be integrally configured. That is to say, the inputter 121 and the display 122 may be configured as a portable terminal (for example, a tablet terminal) having a touch panel, on which direct input operations are performed for various information displayed on the display 122.

The communicator 131 communicates with the culturing system BS and the memory storage device 110 via a network and transmits and receives various information thereto and therefrom. The communicator 131, for example, receives information related to observation conditions and observation results from the culturing system BS. The communicator 131, for example, transmits and receives information related to observation conditions and observation results to and from the memory storage device 110.

The acquirer 132 acquires observation results obtained by image-capturing a plurality of target objects stored in a container having a plurality of storages under predetermined observation conditions. For example, the acquirer 132 appropriately acquires information related to the results of various observations in the culturing system BS stored in the memory storage device 110, from the memory storage device 110 via the network or the communicator 131. The acquirer 132 can appropriately acquire not only information related to observation results but also information related the observation conditions from the memory storage device 110.

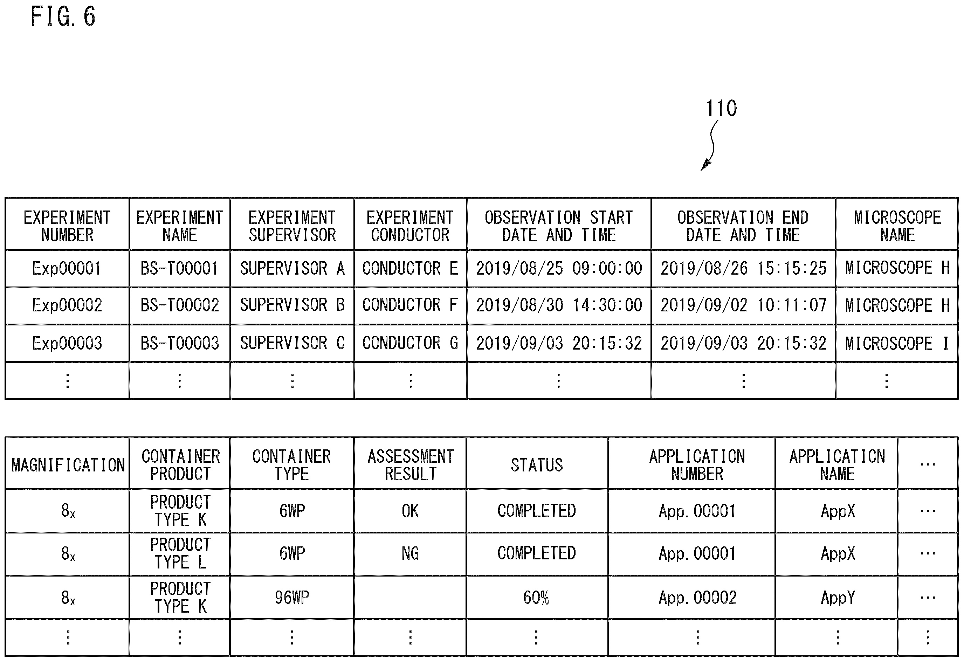

Fig. 6 is a diagram showing an example of information stored in the memory storage device according to the first embodiment. As shown in Fig. 6, the memory storage device 110 includes, as data items, experiment number, experiment name, experiment supervisor, experiment conductor, observation start date and time, observation end date and time, microscope name, magnification, container product, container type, assessment result, status, application number, and application name. The experiment number is information indicating an identification number uniquely assigned to each experiment. For example, the experiment number stores information such as "Exp00001". The experiment name is information indicating the name of an experiment. For example, the experiment name stores information such as "BS-T00001". The experiment supervisor is information indicating the name of a person responsible for an experiment. For example, the experiment supervisor stores information such as "Supervisor A". The experiment conductor is information indicating the name of a person that conducts an experiment. For example, the experiment conductor stores information such as "Conductor E". The data items such as the experiment number, the experiment name, the experiment supervisor, and the experiment conductor may respectively be simply data items such as number, name, supervisor, and conductor. For example, when used not only in an experimental process but also in a culturing process, these data items may be data items such as culturing number, culturing name, culturing supervisor, and culturing conductor.

The observation start date and time is information indicating the date and time on and at which an observation started. For example, the observation start date and time stores information such as "2019/08/25 09:00:00". The observation end date and time is information indicating the date and time on and at which an observation ended. For example, the observation end date and time stores information such as "2019/08/26 15:15:25". The microscope name is information indicating the name of a microscope used for observation. For example, the experiment name stores information such as "Microscope H". The magnification is information indicating the magnification of a microscope set at the time of observation. For example, the magnification stores information such as "8x". The container product is information indicating the manufacturer name of a container (for example, a well plate or the like) having a plurality of storages (for example, wells, dishes, or the like) for storing target objects. For example, the container product stores information such as "Product type K".

The container type is information indicating the type of a container (for example, a well plate or the like) having a plurality of storages (for example, wells, dishes, or the like) for storing target objects. For example, the container type stores information such as "6WP (Well Plate)". The assessment result is information indicating a user assessment of an experiment. For example, the assessment result stores information such as "OK" or "NG". The status is information indicating the analytical progress of an observation result. For example, the status stores information such as "Completed" or "60%". The application number is information indicating an identification number uniquely assigned to each application package used for an observation result analysis. For example, the application number stores information such as "App.00001". The application name is information indicating the name of an application package. For example, the application name stores information such as "AppX". Examples of an application package include an application for analyzing images, an application for calculating the area of a target object, and an application for calculating the number of target objects.

In addition to the information mentioned above, the memory storage device 110 stores an observation image of a plurality of storages, in which target objects are stored respectively, in association with an experiment number, a code number, and so forth. An observation image corresponds to, for example, the overall observation image or microscopic observation image mentioned above. Therefore, the acquirer 132 can also appropriately acquire an observation image from the memory storage device 110. The memory storage device 110 also stores group information described later.

Now, let us return to the description of Fig. 5. The display controller 133 generates a display image to be displayed on the display 122, and displays the generated display image on the display 122. The display controller 133 generates various display images to be displayed on the display 122 and displays the generated display images on the display 122, however, in the present embodiment, the display controller 133 primarily generates a display image related to group registration executed by the registerer 135 described later. The display controller 133 acquires from the calculator 134 information that requires calculation in relation to generation of a display image. That is to say, the calculator 134 executes a calculation on the basis of observation results. In other words, the display controller 133 acquires raw data such as observation conditions and observation results from the memory storage device 110, and acquires from the calculation unit 134 information on calculation processing results based on the observation results. The processing performed by the display controller 133 according to the present embodiment will be described in detail later.

Based on classification criteria obtained from observation conditions and observation results and the observation results, the registerer 135 registers two or more of the storages as the same group. For example, a classification criterion is at least one of the observation conditions including the type and amount of a culture fluid to be charged into the storage. For example, a classification criterion is at least one of the observation conditions including the type, concentration, and amount of a serum included in a culture fluid to be charged into the storage. For example, a classification criterion is at least one of the observation conditions including the type, concentration, exposure duration, and exposure timing of a medicament to be charged into the storage. For example, a classification criterion is at least one of the observation conditions including the type and number of target objects to be charged into the storage. For example, a classification criterion is at least one of the observation conditions including the microscope name, the magnification, temperature setting, humidity setting, atmosphere supply setting, and light output setting in the space in which the container is arranged (for example, culturing chamber 2). For example, a classification criterion is at least one of the observation results including the number of, the temporal change in the number of, the doubling time of the number of, the movement amount of, and the form changes in the target objects. For example, a classification criterion is at least one of the observation results including the area that covers target objects and the perimeter length of the target objects. As a classification criterion, for example, the luminance value after an observation result (image) analysis may be used. For example, in the case where using the luminance value after an image analysis, when the average luminance value is "5" with respect to the cell density (crude density) of a colony and the average value is taken as a reference value, values equal to or greater than the reference value and values less than the reference value can be used for classification. That is to say, the registerer 135 registers two or more storages having the same (similar or related) one or a combination of the above classification criteria and observation result, as the same group. The registerer 135 stores group information in the memory storage device 110. The classification criteria include, for example, a feature or index used for group registration and group display described later.

The information used for group registration may include an observation image of a storage, which is one of observation results. That is to say, the registerer 135 registers two or more storages having the same (similar or related) observation image during any stage (period) of an observation, as the same group. The information used for group registration may include information that visually represents observation results. That is to say, the registerer 135 registers two or more storages having the same (similar or related) information that is graphically represented information on the basis of the information graphically representing the observation result, as the same group.

Group registration can also be performed without using observation results. For example, the registerer 135 executes group registration at an arbitrary timing without using observation results. Examples of the arbitrary timing include before, during, and/or after an observation. As an example, group registration that does not use observation results can be performed before an observation (at a timing at which classification criteria related to observation conditions are set). That is to say, based on classification criteria related to observation conditions, the registerer 135 may register two or more of the storages as the same group. The classification criteria related observation conditions may be predetermined by the user.

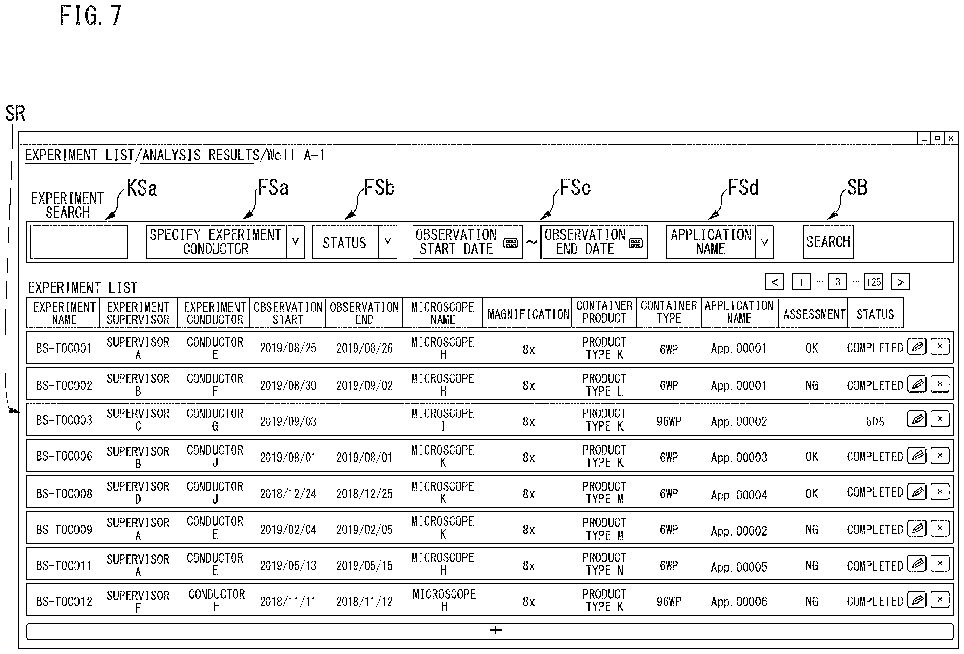

Fig. 7 to Fig. 16 are diagrams each showing a screen example in a group registration that uses classification criteria according to the first embodiment. In the description of Fig. 7 to Fig. 16, the processes in the display controller 133, the calculator 134, and the registerer 135 will be described as appropriate. As shown in Fig. 7, the display controller 133 displays a display image showing an observation result search screen on the display 122. The observation result search screen includes a text input field KSa for performing keyword search, conditional search fields FSa, FSb, FSc, and FSd for performing a search by making a selection from predetermined search conditions, and a search button SB for executing a search. For example, the condition search field FSa is a pull-down menu for selecting the name of an experiment conductor. For example, the condition search field FSb is a pull-down menu for selecting a status. For example, the condition search field FSc has pull-down menus for selecting an observation start date and time and an observation end date and time. For example, the condition search field FSd is a pull-down menu for selecting an application name. The conditional search fields FSa, FSb, FSc, and FSd may be realized by text input fields. The search conditions are not limited to the above examples.

The user operates the inputter 121, inputs text to the text input field KSa, makes selections using the conditional search fields FSa, FSb, FSc, and FSd, and presses the search button SB. Alternatively, the user operates the inputter 121 and presses the search button SB without performing text input or making search condition selections. Upon the search button SB having been pressed, the display controller 133 acquires information corresponding to the search conditions from the memory storage device 110, and displays a display image showing a search result field SR on the display 122. That is to say, the display controller 133 displays on the display 122 a display image showing a list of observation results based on the search conditions or a list of all observation results. For example, the search result field SR includes information on data items such as experiment name, experiment supervisor, experiment conductor, observation start date and time, observation end date and time, microscope name, magnification, container product, container type, application name, assessment, and status. However, data items of the search result field SR are not limited to the above examples. Sorting can be performed in the search result field SR, using a data item instruction or the like. The user performs an operation of making a selection (specifying) from the search result field SR in order to confirm an observation result.

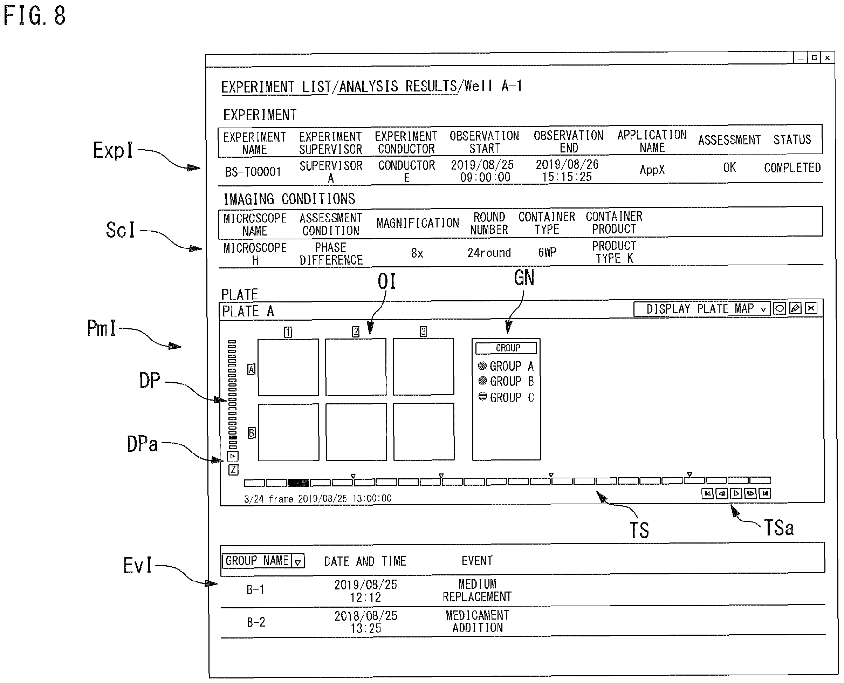

Next, as shown in Fig. 8, the display controller 133 shows on a display image the observation result selected from the search result field SR. The display image of the observation result includes, for example, an information field ExpI related to observation, an information field ScI related to image capturing conditions (observation conditions), a plate map field PmI including observation images, and an information field EvI related to events in observations. Of these, the plate map field PmI includes observation image fields OI, a group name field GN, a time-series switching content field TS, and a depth switching content field DP. For example, each observation image field OI displays an observation image included in a selected observation result. For example, the group name field GN displays registered group names. For example, the time-series switching content field TS displays contents for switching observation images in time series manner. For example, the depth switching content field TS displays contents for switching observation images corresponding to the depths of storages.

The time-series switching content field TS is represented by, for example, a plurality of rectangles separated at regular intervals. The user can operate the inputter 121 to select a rectangle in the time-series switching content field TS. When a rectangle in the time-series switching content field TS is selected, the display controller 133 switches the display of the plate map field PmI using the observation image corresponding to the corresponding period as a display image. At this time, the display controller 133 may display information related the observation date and time of the selected rectangle. Switching of observation images using the time-series switching content field TS is not limited to selecting a rectangle, and may be realized by a time-series switching content field TSa that moves the position of the selected rectangle.

The depth switching content field DP is represented by, for example, rectangles separated at certain depths (thicknesses in the Z direction) with respect to the storage. The user can operate the inputter 121 to select a rectangle in the depth switching content field DP. When a rectangle in the depth switching content field DP is selected, the display controller 133 switches the display of the plate map field PmI, using the observation image corresponding to the corresponding depth of the storage as a display image. Switching of observation images using the depth switching content DP is not limited to selecting a rectangle, and may be realized by a depth switching content field DPa that moves the position of the selected rectangle.

That is to say, the display controller 133 shows the observation results of the group selected by the user, on a display image related to a container having a plurality of storages, and displays the display image on the display 122. When no search condition is used, the display controller 133 shows observation results selected from a list (a list of all results) by the user, in a display image related to a container having a plurality of storages, and displays the display image on the display 122. When search conditions are used, the display controller 133 shows observation results selected by the user from a list based on the search conditions, in a display image related to a container having a plurality of storages, and displays the display image on the display 122. The display controller 133 shows the observation images of the storages included in the observation results, in the display image, and displays the display image on the display 122.

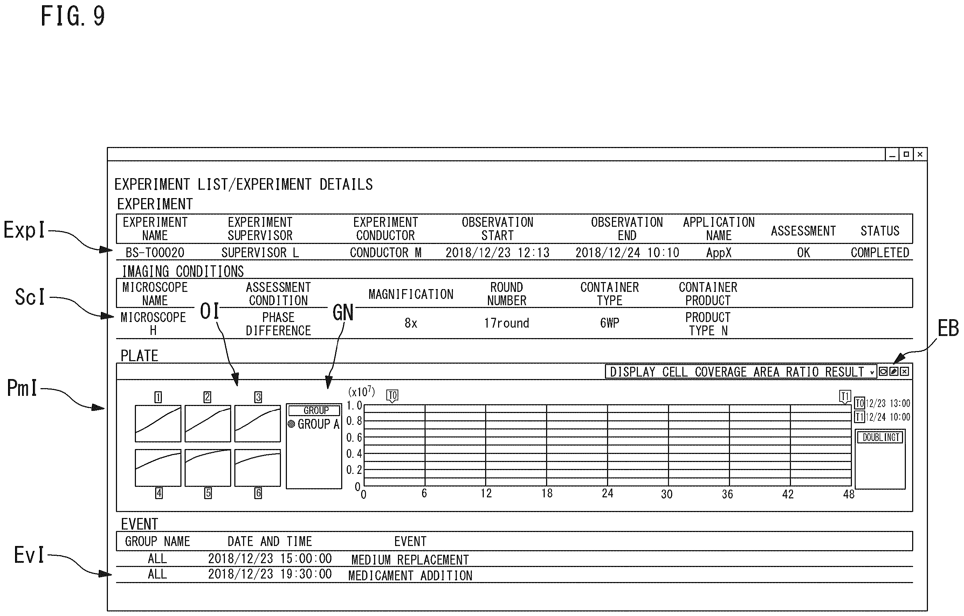

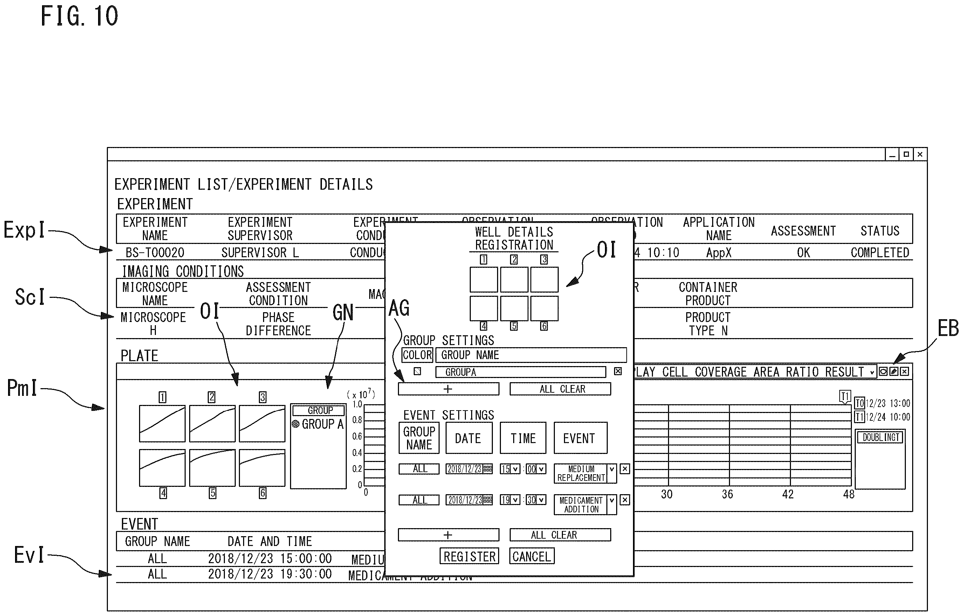

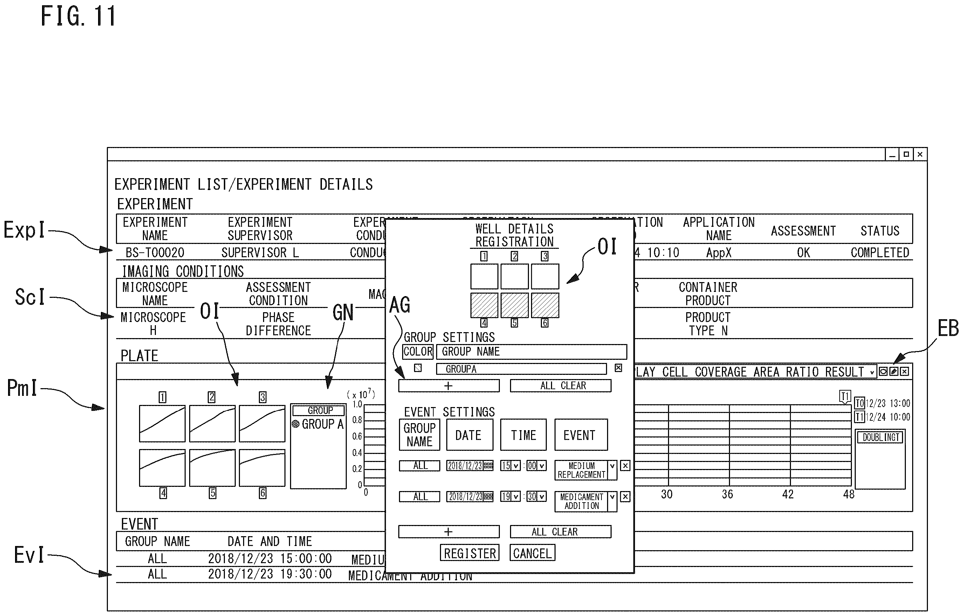

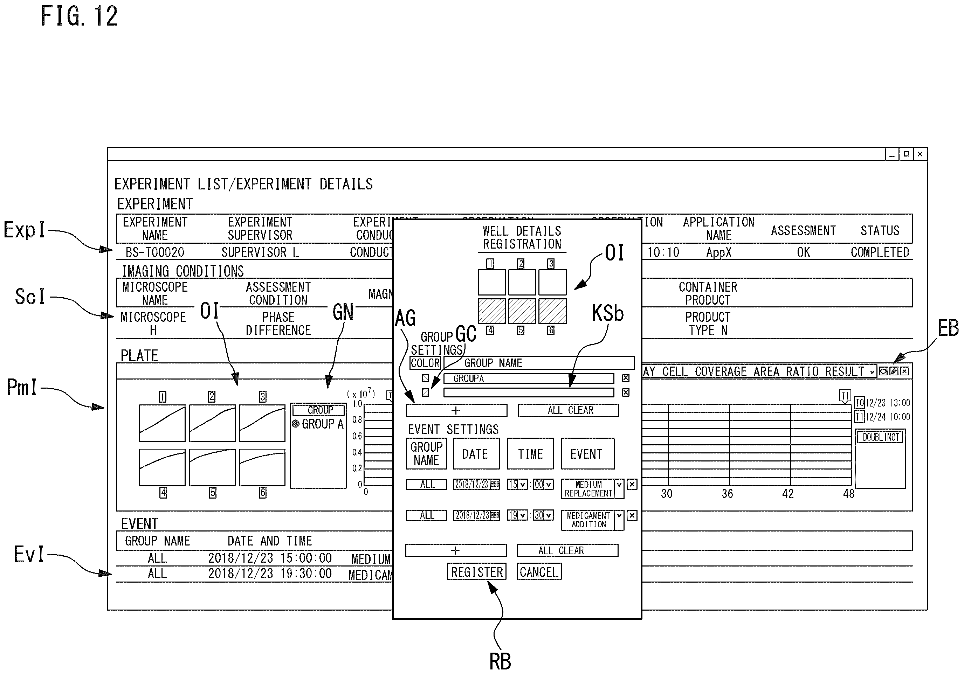

Next, the user operates the inputter 121 and presses an edit button EB (see Fig. 9) for a group registration. Here, editing includes editing group information by registering a new group. As shown in Fig. 9, the display controller 133 accepts a signal indicating that the edit button EB has been pressed. Then, as shown in Fig. 10, the display controller 133 displays a display image including observation image fields OI and a group add button AG for adding a new group. Next, as shown in Fig. 11, the user operates the inputter 121, selects two or more observation image fields OI corresponding to the storages to be added to the new group, and presses the group add button AG. Here, the display controller 133 displays a display image in which the observation image field OI is inverted with a predetermined color according to the selected the observation image field OI. As a result, the display controller 133 receives a signal indicating the group add button AG having been pressed, and as shown in Fig. 12, the display controller 133 displays a display image including a text input field KSb for inputting (or editing) a group name for the new group and a register button RB for executing group registration. In addition, the display controller 133 highlights the observation image fields OI of the group registration target, using emphasizing information such as a color, a frame, and lines. In the vicinity of the text input field KSb there is arranged a group color content field GC that represents observation results and so forth in a different color for each group.

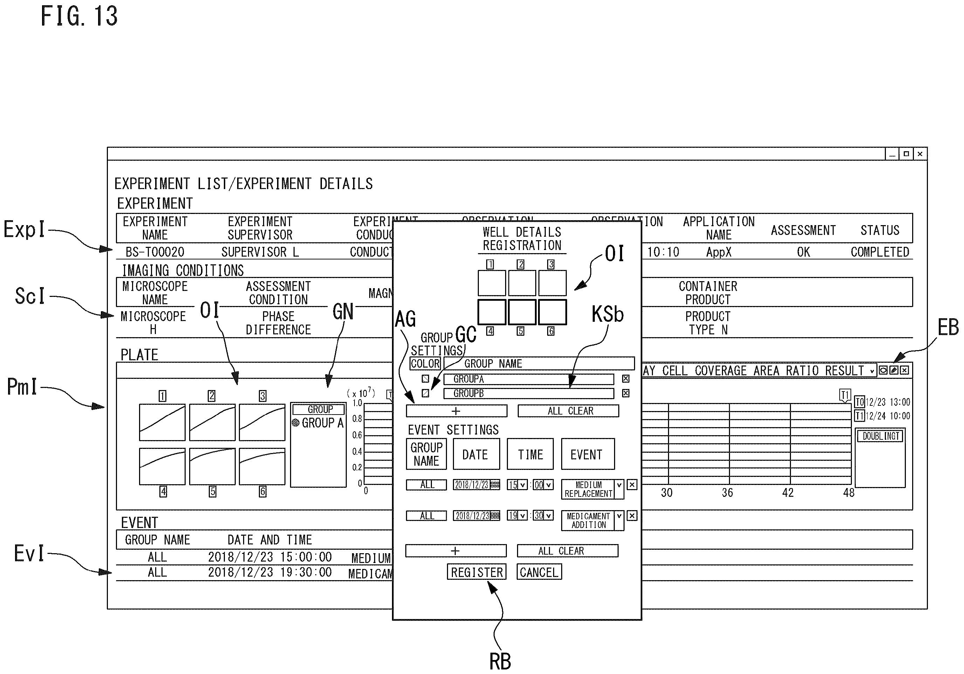

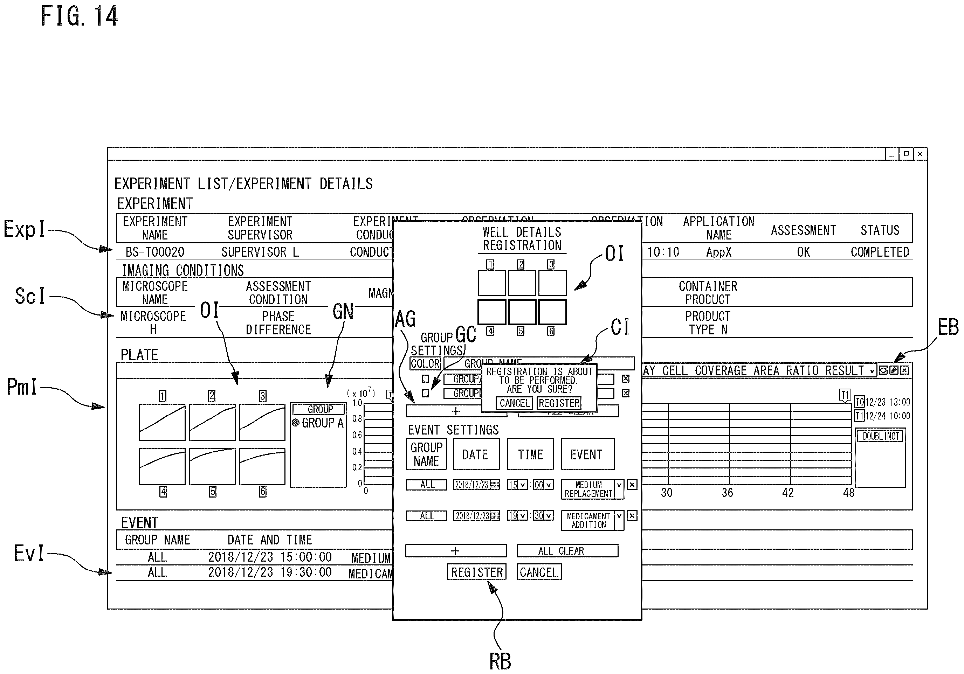

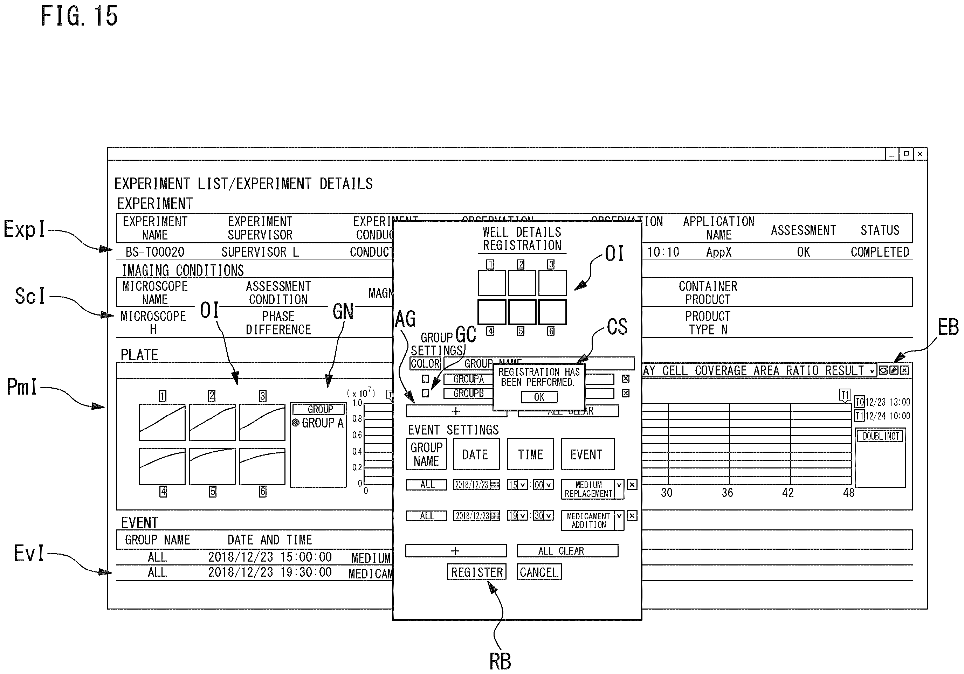

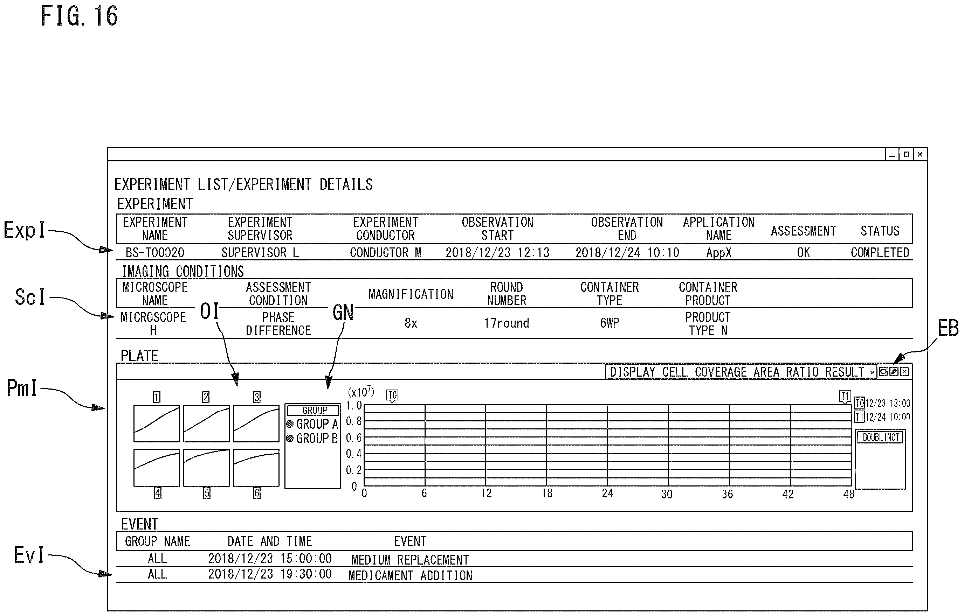

Next, as shown in Fig. 13, the user operates the inputter 121, makes a selection in the group color content field GC, inputs a group name to the text input field KSb, and presses the register button RB. As a result, the display controller 133 receives a signal indicating the register button AG having been pressed, and as shown in Fig. 14, the display controller 133 displays a display image including a confirmation image CI showing an image that confirms the execution of group registration. When executing group registration using classification criteria, the user operates the inputter 121 and presses the registration button included in the confirmation image CI. On the other hand, when not executing group registration, the user operates the inputter 121 and presses a cancel button included in the confirmation image CI. As shown in Fig. 15, when the register button is pressed, the display controller 133 displays a display image including a completion image CS indicating the completion of the group registration. At this time, the registerer 135 stores group information related to the new group in the memory storage device 110. After that, as shown in Fig. 16, the display controller 133 displays a display image including the newly registered group name in the group name field GN in the plate map field PmI. The information processing device 100 completes the group registration as described above.

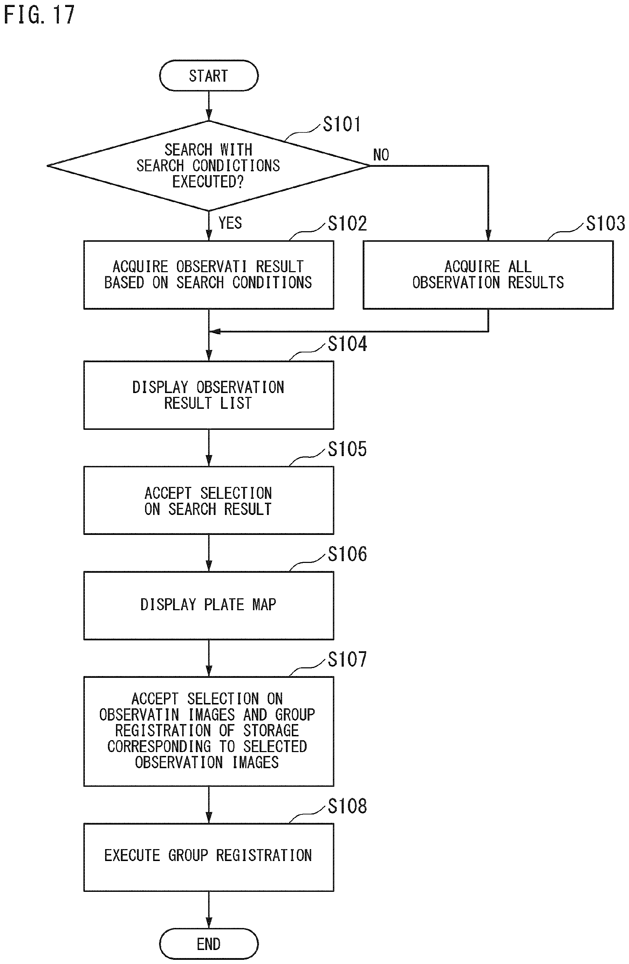

Fig. 17 is a flowchart showing an example of a process flow in the information processing device according to the first embodiment. In Step S101, the acquirer 132 determines whether a search has been executed with search conditions set therefor. For example, the acquisition unit 132 determines which one of the following types of searches has been executed. A search executed by inputting text in the text input field KSa and then pressing the search button SB; a search executed by selecting search conditions using the condition search fields FSa, FSb, FSc, and FSd and then pressing the search button SB; and a search executed only by pressing the search button SB. At this time, if the executed search is a search executed by inputting text in the text input field KSa and then pressing the search button SB or a search executed by selecting search conditions using the condition search fields FSa, FSb, FSc, and FSd and then pressing the search button SB (Step S101: Yes), the acquirer 132 executes the processing of Step S102. On the other hand, if the executed search is a search executed only by pressing the search button SB (Step S101: No), the acquirer 132 executes the process in Step S103.

In Step S102, the acquirer 132 acquires observation results based on the search conditions. For example, the acquirer 132 acquires observation results corresponding to the search conditions from the memory storage device 110 via the communicator 131. In Step S103, the acquirer 132 acquires all observation results. For example, the acquirer 132 acquires all observation results from the memory storage device 110 via the communicator 131. In Step S104, the display controller 133 displays a list of observation results. For example, the display controller 133 shows a list of observation results acquired by the acquirer 132 in a display image, and displays the display image on the display 122.

In Step S105, the display controller 133 accepts a selection of an observation result. For example, on the display image of an observation result list, the display controller 133 accepts the user's selection of an observation result as a signal. In Step S106, the display controller 133 displays a plate map. For example, the display controller 133 shows the observation result selected by the user, in a display image related to a container having a plurality of storages, and displays the display image on the display 122. At this time, the display controller 133 may show a graph or the like for the observation result in a display image on the basis of the result of calculation executed by the calculator 134, and display the display image on the display 122. The display controller 133 may show the time-series switching content field TS and the depth switching content field DP in a display image, and display the display image on the display 122.

In Step S107, the display controller 133 accepts a selection of observation images and a group registration of the storage corresponding to the selected observation images. For example, the display controller 133 accepts a signal indicating that the edit button EB has been pressed. The display controller 133 then displays on the display 122 a display image including the observation image fields OI and the group add button AG. Next, the display controller 133 accepts a signal indicating that a selection has been made from the observation image fields OI and the group add button AG has been pressed. At this time, the display controller 133 displays a display image in which the selected observation image field OI is inverted with a predetermined color. After that, the display controller 133 displays on the display 122 a display image including the text input field KSb for inputting a group name and the register button RB for executing the group registration. At this time, the observation image field OI of the group registration target may be highlighted a frame. Then, the display controller 133 accepts a signal indicating that a selection has been made in the group color content field GC, a group name has been input in the text input field KSb, and the register button RB has been pressed.

In Step S108, the registerer 135 executes the group registration. For example, for the target observation results of the new group, the registerer 135 stores group information in the memory storage device 110 on the basis of the group color and the group name accepted by the display controller 133. Group registration is not limited to a new group registration. For example, as shown in Fig. 11, after selecting the observation image field OI, the user may select an existing group (here, "Group A") and press the registration button RB. As a result, the registerer 135 may store in the memory storage device 110 the group information to be added to the "Group A" for the selected storages.

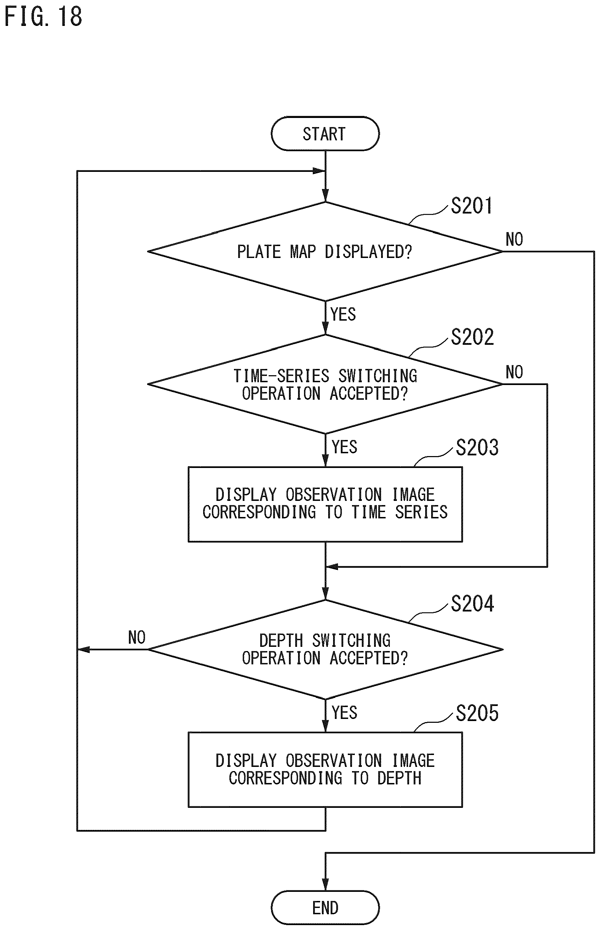

Fig. 18 is a flowchart showing an example of a flow of a process for switching observation image display according to the first embodiment. In Step S201, the display controller 133 determines whether a plate map is being displayed. At this time, if a plate map is being displayed (Step S201: Yes), the display controller 133 executes the process of Step S202. On the other hand, if a plate map is not being displayed (Step S201: No), the display controller 133 ends the process because switching of observation image display does not need to be executed.

In Step S202, the display controller 133 determines whether a time-series switching operation has been accepted. For example, the display controller 133 determines whether a rectangle in the time-series switching content field TS is selected. The display controller 133 may determine whether an operation of moving the position of selected rectangle has been performed in the time-series switching content field TSa. Then, if a signal indicating a time-series switching operation has been accepted (Step S202: Yes), the display controller 133 displays an observation image corresponding to the time-series in Step S203. For example, the display controller 133 switches the display of the plate map field PmI, where the observation image corresponding to the period that corresponds to the position of the rectangle in the time-series switching content field TS serves as a display image. On the other hand, if a time-series switching operation has not been accepted (Step S202: No), the display controller 133 executes the process of Step S204.

In Step S204, the display controller 133 determines whether a depth switching operation has been accepted. For example, the display controller 133 determines whether a rectangle in the depth switching content field DP is selected. The display controller 133 may determine whether an operation of moving the position of selected rectangle has been performed in the depth switching content field DPa. If a signal indicating a depth switching operation has been accepted (Step S204: Yes), the display controller 133 displays an observation image corresponding to the depth in Step S205. For example, when a rectangle in the depth switching content field DP is selected, the display controller 133 switches the display of the plate map field PmI using the observation image corresponding to the corresponding depth of the storage as a display image. On the other hand, if a depth switching operation has not been accepted (Step S204: No), the display controller 133 executes the process of Step S201. That is to say, in the case where a signal indicating an operation in the time-series switching content field TS or in the depth switching content field DP is accepted while the plate map is displayed, the display controller 133 executes the process of switching to and displaying the corresponding observation image.

### Second Embodiment

Hereunder, a second embodiment will be described. In the present embodiment, the same reference signs are given to configurations similar to those in the embodiment described above, and the descriptions thereof may be omitted or simplified in some cases.

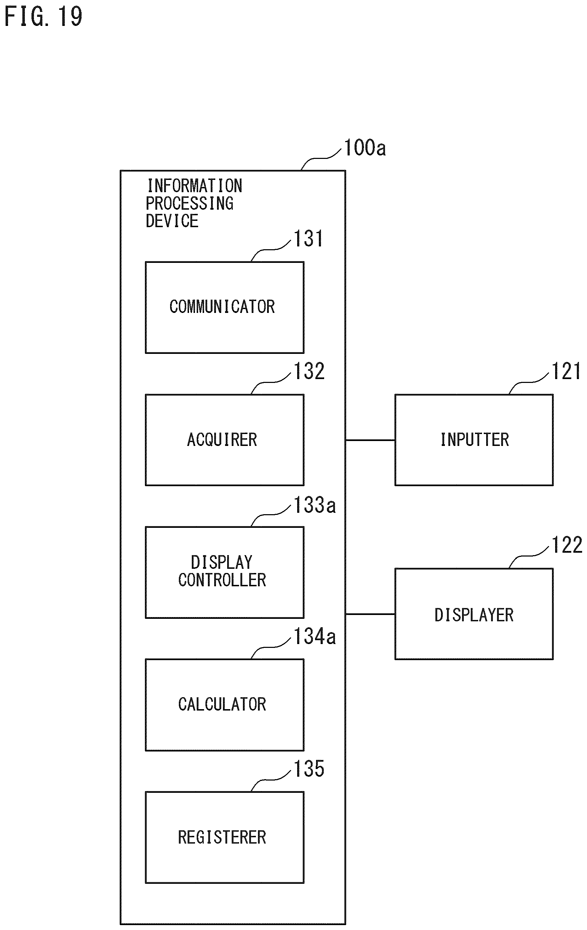

Fig. 19 is a block diagram showing a functional configuration example of an information processing device according to the second embodiment. As shown in Fig. 19, an information processing device 100a has a communicator 131, an acquirer 132, a display controller 133a, a calculator 134a, and a registerer 135. An inputter 121 and a display 122 are connected to the information processing device 100a. The display controller 133a corresponds to a "controller". The calculator 134a corresponds to a "time calculator".

The acquirer 132 acquires observation results obtained from a time-lapse image of a target object. As described above, a time-lapse image of a target object is an image based on overall observation images and microscopic observation images, and is an image generated by the control unit 6, for example. The process in the acquirer 132 is similar to that in the embodiment described above. The display controller 133a indicates, in a display image, analysis results that visually represent observation results and an increase/decrease time that is calculated on the basis of observation results and that indicates the amount of time required to increase or decrease a predetermined value related to the target object. When a setting for a section of time (for example, a signal indicating a user input) has been received, the display controller 133a indicates, in the display image, the increase/decrease time that corresponds to the section that has been set. The section mentioned above in the present embodiment is, for example, a set of points (times in this case) that includes a time between a certain time and another time, and that is made up of real numbers, and it includes at least any two points in the set.

The increase/decrease time is, for example, information indicating an amount of time required for the number of target objects to increase or decrease to a predetermined value at a given time, and includes a doubling time. The predetermined value can be set by the user according to the type of the observation target object. In the present embodiment, a doubling time will be described as an example of the increase/decrease time. The doubling time is found by, for example, "T_{DT} = ln· [ (t₁-t₂) / (y₁-y₂)] ". T_{DT} indicates a doubling time, and ln indicates a natural logarithm (logₑ2). t₁ indicates a time at the start position of the section, and t₂ indicates a time at the end position of the section. y₁ indicates the number of target objects at the start position of the section, and y₂ indicates the number of target objects at the end position of the section.

That is to say, the display controller 133a has the number of target objects on the vertical axis and time on the horizontal axis to indicate, in a display image, both analysis results visually representing the observation results in a graph and the doubling time of the target objects based on the setting of the section of time in the graph (for example, the section between time T1 and time T20, time T5 and time T10, etc., after cell culturing), and displays the display image on the display 122. The vertical axis of the graph is not limited to the number of target objects. The display controller 133a may, for example, have the area of target objects on the vertical axis to indicate, in a display image, analysis results in which observation results are rendered in a graph. The display controller 133a may, for example, have the perimeter length of target objects on the vertical axis to indicate, in a display image, analysis results in which observation results are rendered in a graph. Upon receiving a signal indicating a change of the section of time in a graph that visually represents information, the display controller 133a displays in the display image the calculated doubling time corresponding to the section that has been received, and displays on the display 122 the display image including the graph, in which the section has been changed, and the doubling time corresponding to the changed section. Setting (for example, performing initial setting, or changing setting) of a section of time in the graph is realized by the user performing a user operation while the user visually confirming the graph (image) displayed as part of the display image. That is to say, at the timing of the user operation, if a signal indicating a change of the section of time in the graph is received, the display controller 133a indicates in the display image the doubling time corresponding to the changed section. The processing related to the display of a display image according to the present embodiment will be described in detail below, with reference to the drawings.

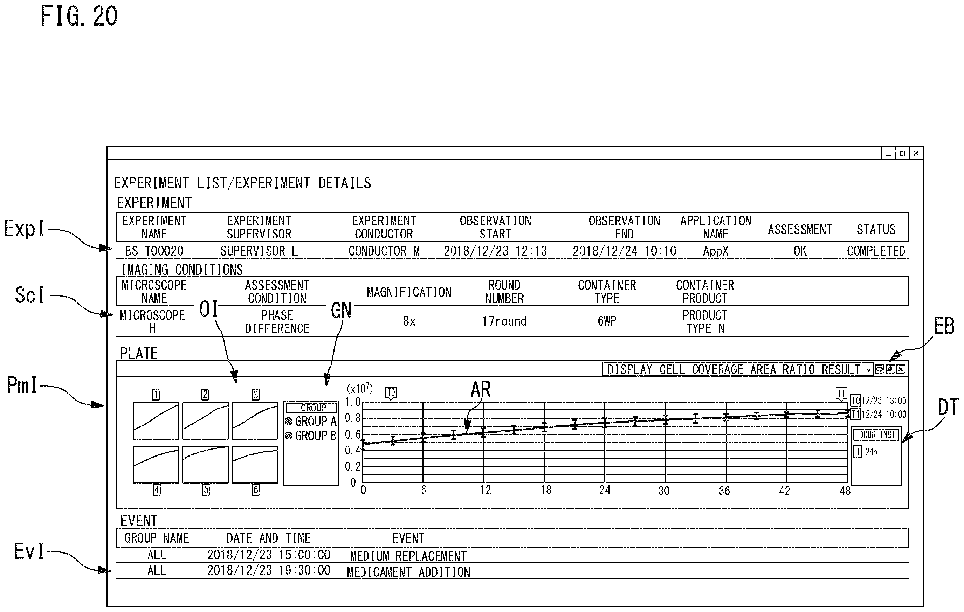

Fig. 20 to Fig. 23 are diagrams each showing an example of a display image including an analysis result and a doubling time according to the second embodiment. In Fig. 20, as an example, the screen after group registration will be described. The user operates the inputter 121 and selects a registered group from the group name field GN in the plate map field PmI. As a result, as shown in Fig. 20, the display controller 133a displays on the display 122 a display image including an analysis result field AR that indicates the analysis result corresponding to the group selected from a plurality of group names displayed as an observation results list by the user and the doubling time field DT that indicates a doubling time. For example, when displaying the analysis result field AR and the doubling time field DT, the display controller 133a acquires a preliminarily calculated doubling time from the memory storage device 110. That is to say, the doubling time may be preliminarily calculated at the timing of storing observation results in the memory storage device 110. Thus, the calculation of doubling time in the present embodiment may be performed preliminarily at the timing of storing observation results in the memory storage device 110, or may be performed at the timing of receiving the signal indicating the change of the section mentioned above.

The display controller 133a may accept a calculation processing result from the calculator 134a and display a doubling time. That is to say, the calculator 134a may calculate a doubling time in a real-time manner when displaying the analysis result field AR. When the user selects another group, the display controller 133a shows a display image that has switched to the analysis result of the corresponding group according to the selection made by the user, and displays the display image on the display 122. At this time, the display controller 133a also switches and displays the doubling time. The display controller 133a can show the graph and the doubling time in a display image so as to fit on one screen (for example, one screen of the browser) and the display image is displayed on the display 122. The analysis result field AR shown in Fig. 20 is an example of a visually representing the average number of target objects in the storages belonging to a group B, together with error bars.

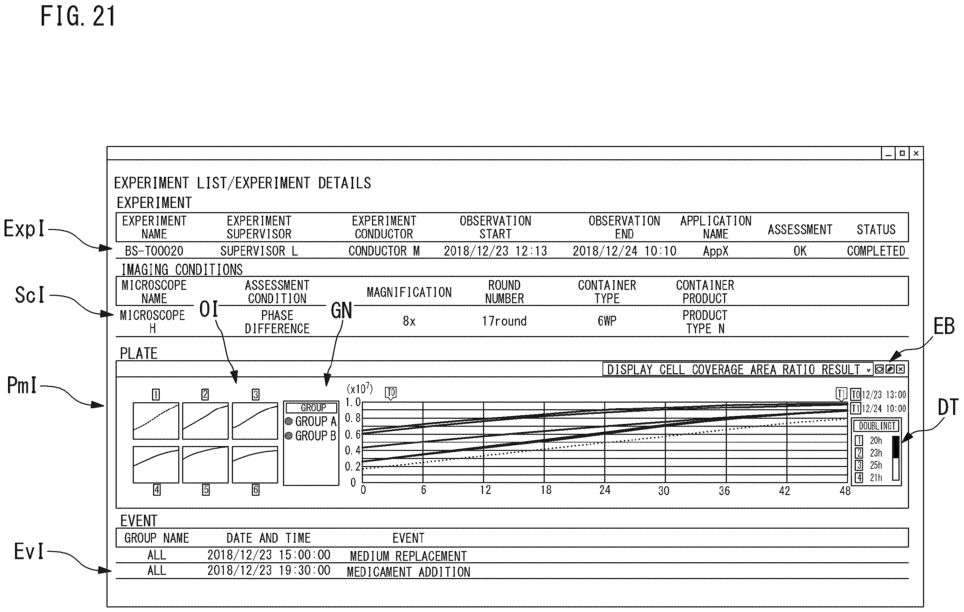

Unlike Fig. 20, the number of target objects in each of the storages belonging to one group may be visually represented. In such a case, as shown in Fig. 21, the analysis result field AR displays a number of graphs corresponding to the number of storages. The doubling time field DT is displayed for each of the graphs. The user can use the scroll bar to confirm the doubling time field DT that does not fit in the display region.

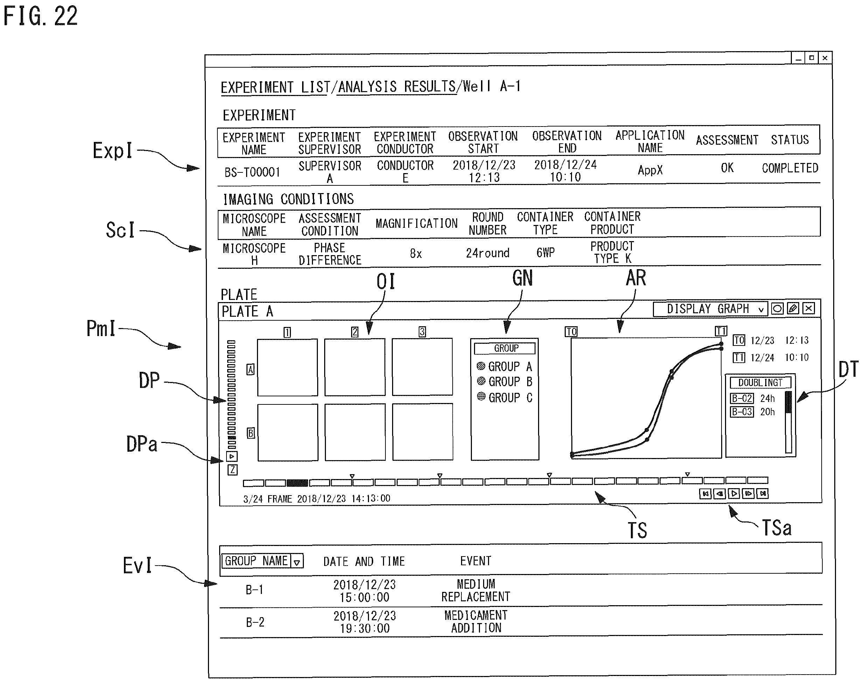

Unlike Fig. 20 and Fig. 21, the average number of target objects in a storage that belongs to a plurality of groups may each be visually represented. In such a case, as shown in Fig. 22, the analysis result field AR displays in each graph the average number of target objects in each storage that belongs to a plurality of selected groups. The doubling time field DT is displayed for each graph. That is to say, the display controller 133a displays, in a display image, a graph as an analysis result corresponding to each of the plurality of groups selected by the user from the plurality of group names displayed as an observation results list, and displays the display image on the display 122.

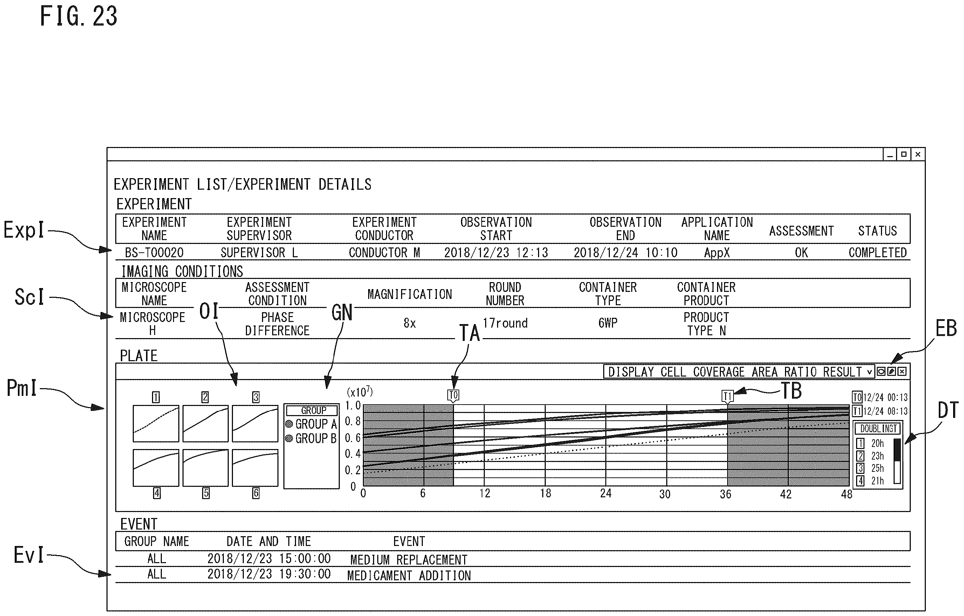

The section of time in the analysis result may be set, using section-setting contents (for example, two moving bar icons). As shown in Fig. 23, the display controller 133a indicates section-setting contents TA, TB for setting a section of time in the analysis result on the graph of the display image. The section-setting content TA is a content that sets the time at the start position of the section. In Fig. 23, the section-setting content TA is represented by "T0". T0 corresponds to a time "t₁" used in a doubling time calculation. The section-setting content TB is a content that sets the time at the end position of the section. In Fig. 23, the section-setting content TB is represented by "T1". T1 corresponds to a time "t₂" used in the doubling time calculation. The user sets the section by moving each of the section-setting contents TA, TB to an arbitrary position on the time axis. That is to say, the display controller 133a indicates the section-setting contents for setting the section in the display image, so as to be movable in the direction of a time axis in the analysis results, and when the section-setting contents are moved by a user operation, the display controller 133a receives the change of the section and displays in the display image the doubling time corresponding to the section that has been received.

The display controller 133a indicates, in the display image, information related to the date and time of the section. Fig. 23 gives an example of displaying, above the doubling time field DT, the date and time at the start position of the section and the date and time at the end position of the section. That is to say, when the section-setting contents TA, TB are moved by the user operation, according to the movement thereof, the display controller 133a switches the information related to the date and time of the section that has been setand indicates it in the display image, and the display controller 133a displays the display image on the display 122. The display controller 133a indicates, in the display image, different representational effects inside and outside the section set by the section-setting contents TA, TB. As a representational effect for hiding the regions outside the section, Fig. 23 shows an example in which the user who operates to move the section-setting contents TA, TB can clearly recognize the section.

In the graph of the present embodiment (for example, the location where the analysis result field AR is displayed), data on the vertical axis and the horizontal axis of the graph are shown as an example. For example, the horizontal axis may display information related to times during a period from an observation start date and time to an observation end date and time. For example, vertical axis may display information for an item selected from the pull-down menu in the plate map field PmI. For example, when the number of cells is selected in the pull-down menu, the vertical axis represents values in a range such as 0 to 1.0 (×10⁷). For example, when the cell coverage area ratio is selected in the pull-down menu, the vertical axis represents ratios in a range such as 0 to 100 (or 0 to 1.0). Therefore, the vertical axis and the horizontal axis of the graph change, depending on the displayed content.

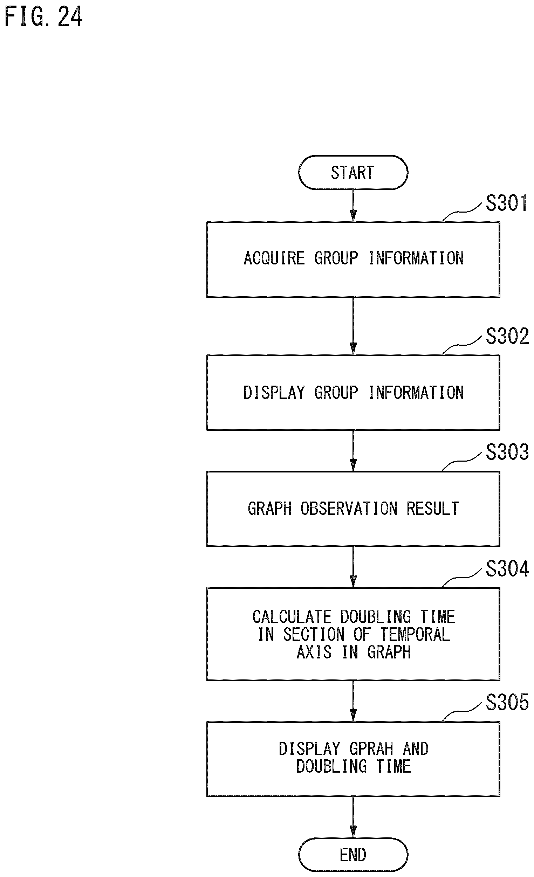

Fig. 24 is a flowchart showing an example of a process flow in the information processing device according to the second embodiment. In Step S301, the acquirer 132 acquires from the memory storage device 110 information related to observation results of each group. In Step S302, the display controller 133a displays on the display 122 a display image including the information related to the observation results of each group acquired by the acquirer 132. Here, the user operates the inputter 121 to select an arbitrary group. The user may select a plurality of groups or select one group only.

In Step S303, the acquirer 132 acquires from the memory storage device 110 observation results that belong to the group selected by the user. The display controller 133a then renders the observation results acquired by the acquirer 132 into a graph. In Step S304, the calculator 134a calculates the doubling time in the section on the time axis of the graph. The display controller 133a may use a doubling time that is preliminarily stored in the memory storage device 110. In Step S305, the display controller 133a displays on the display 122 a display image including the graph and the doubling time.

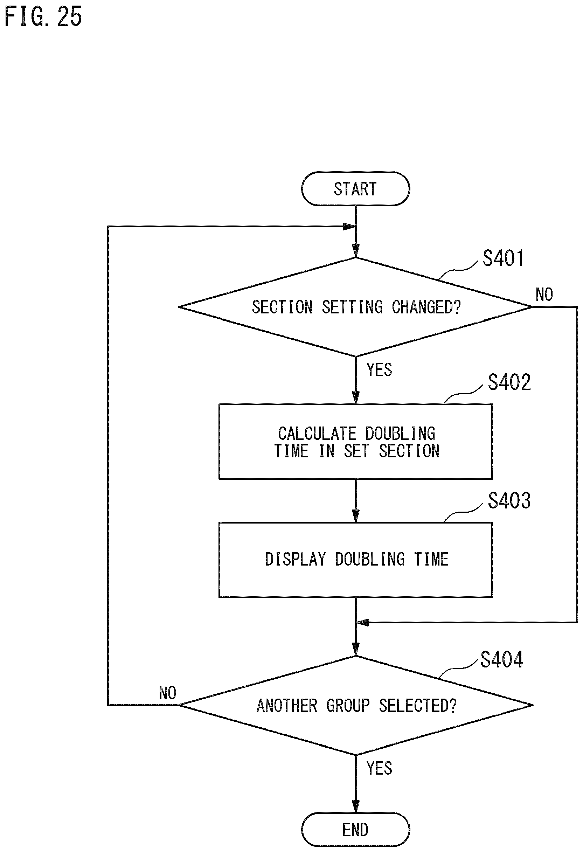

Fig. 25 is a flowchart showing an example of a flow of a process of displaying a doubling time corresponding to a setting of a section according to the second embodiment. In Step S401, the calculator 134c determines whether the section setting in the graph has been changed. At this time, if the section setting has been changed (Step S401: Yes), the calculator 134a calculates, in Step S402, the doubling time in the section that has been set. The display controller 133a may use a doubling time (doubling time according to the section setting change having been made) that is preliminarily stored in the memory storage device 110. On the other hand, if the section setting has not been changed (Step S401: No), the process of Step S404 is executed.

In Step S403, the display controller 133a displays on the display 122 a display image including the doubling time calculated by the calculator 134a. In Step S404, the display controller 133a determines whether another group has been selected. At this time, if another group has been selected (Step S404: Yes), the display controller 133a ends the process of displaying the doubling time corresponding to the analysis result that has been displayed. On the other hand, if another group has not been selected (Step S404: No), the process of Step S401 is executed.

### [Third Embodiment]

Hereunder, a third embodiment will be described. In the present embodiment, the same reference signs are given to configurations similar to those in the embodiment described above, and the descriptions thereof may be omitted or simplified in some cases.

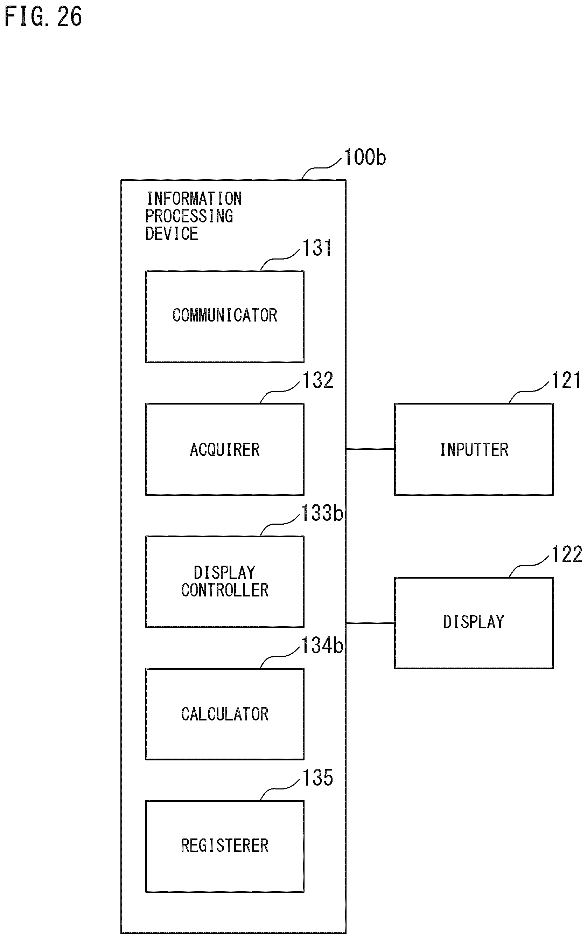

Fig. 26 is a block diagram showing a functional configuration example of an information processing device according to the third embodiment. As shown in Fig. 26, an information processing device 100b has a communicator 131, an acquirer 132, a display controller 133b, a calculator 134b, and a registerer 135. An inputter 121 and a display 122 are connected to the information processing device 100b. The display controller 133b corresponds to a "controller". The calculator 134b corresponds to a "time calculator" and a "normalization calculator".

The display controller 133b indicates, in a display image, analysis results normalized on the basis of a section. For example, in a situation where analysis results based on a section setting using the section-setting contents TA, TB are being displayed, the display controller 133b accepts an instruction to normalize the analysis results upon a user operation. Upon having accepted the normalization instruction, the display controller 133b acquires from the memory storage device 110 the result of normalization calculation performed on the basis of the section that has been set. Then, the display controller 133b indicates, in a display image, a graph serving as an analysis result normalized on the basis of the acquired calculation result, and displays the display image on the display 122. The calculator 134b may calculate the result of normalization calculation performed on the basis of the section that has been set, where appropriate. That is to say, in the case where an instruction to normalize the analysis result is accepted upon a user operation, the calculator 134b executes a calculation related to the normalization of the analysis result. As a result, the display controller 133b indicates, in a display image, a graph serving as an analysis result normalized on the basis of the calculation result of the calculator 134b, and displays the display image on the display 122. The processing related to the display of a display image including the normalized analysis result according to the present embodiment will be described in detail below, with reference to the drawings.

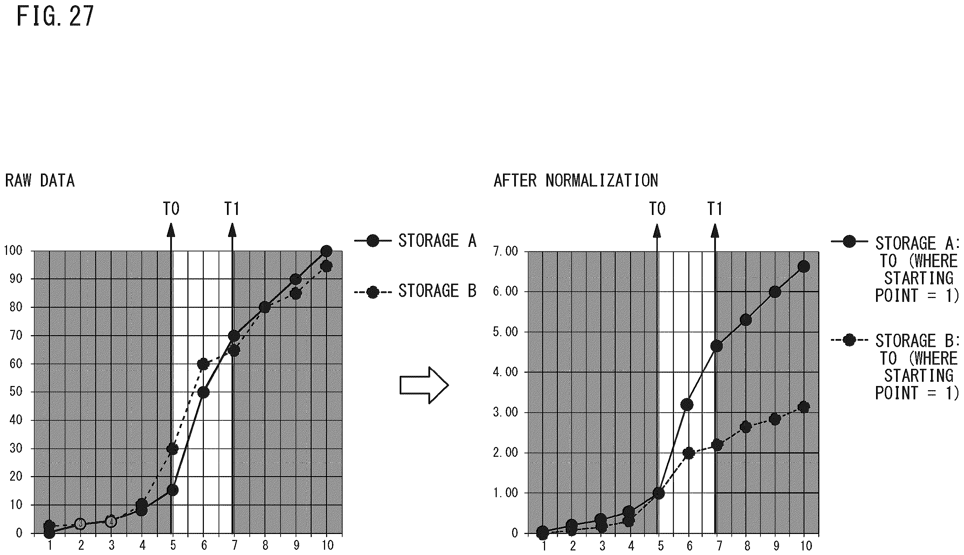

Fig. 27 is a diagram for describing an example of normalization of analysis results according to the third embodiment. In Fig. 27, analysis results (graphs) corresponding to the number of target objects in two storages will be described as an example. As shown on the left side of Fig. 27, when the number of target objects in each storage included in the observation result is plotted on a graph, it is difficult to see which one of the storage A and the storage B is culturing better than the other (for example, which one is better in terms of cell maturity or cell differentiation). For example, the number of target objects in the storage B is greater than the number of target objects in the storage A up until near the halfway between of the start and end of the section, whereas the number of target objects in the storage A becomes greater than the number of target objects in the storage B near the end of the section. The user thus instructs to perform normalization based on the section that has been set. As a result, as shown on the right side of Fig. 27, in the normalized analysis result, the numbers of target objects in the storage A and the storage B are represented without being reversed. That is to say, in the example shown in Fig. 27, it can be seen that the storage A is culturing better than the storage B.

As an example, normalization can be calculated as follows. For example, the entire graph is normalized where the value at the section start T0 takes "1.00" (as reference). Let the value (number of target objects) at T0 of the storage A be "30", the next value be "60", and the value at T1 be "65". The calculator 134b performs calculation as "60 ÷ 30 = 2.00", "65 ÷ 30 = 2.17", and so forth for the analysis result of the storage A. Meanwhile, let the value (number of target objects) at T0 of the storage B be "15", the next value be "50", and the value at T1 be "70". The calculator 134b performs calculation as "50 ÷ 15 = 3.33", "70 ÷ 15 = 4.67", and so forth for the analysis result of the storage B. The calculator 134b executes the above calculation for all values. After performing normalization, the scale of the vertical axis also changes. As a result, the user can easily recognize that the storage A is culturing better than the storage B.

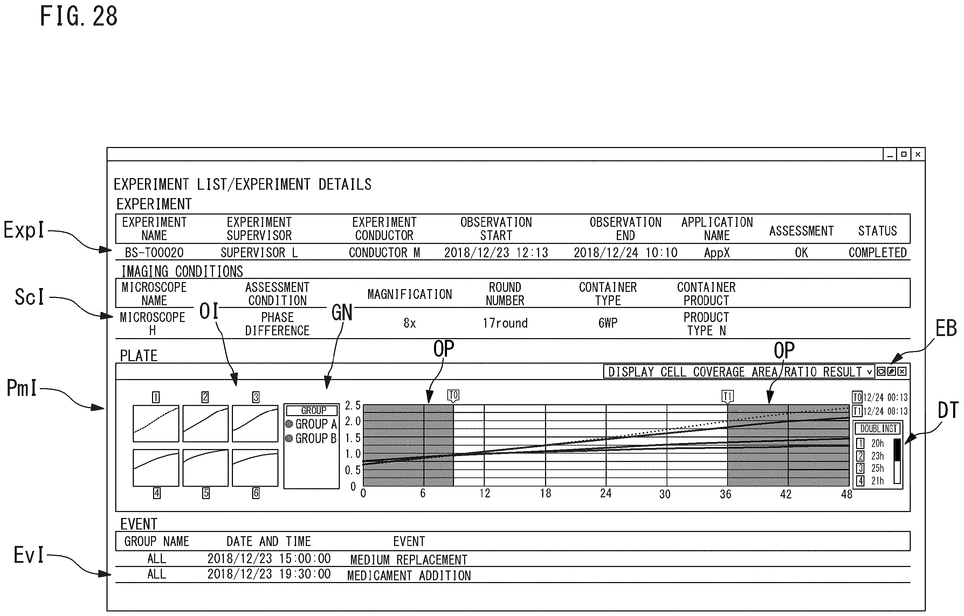

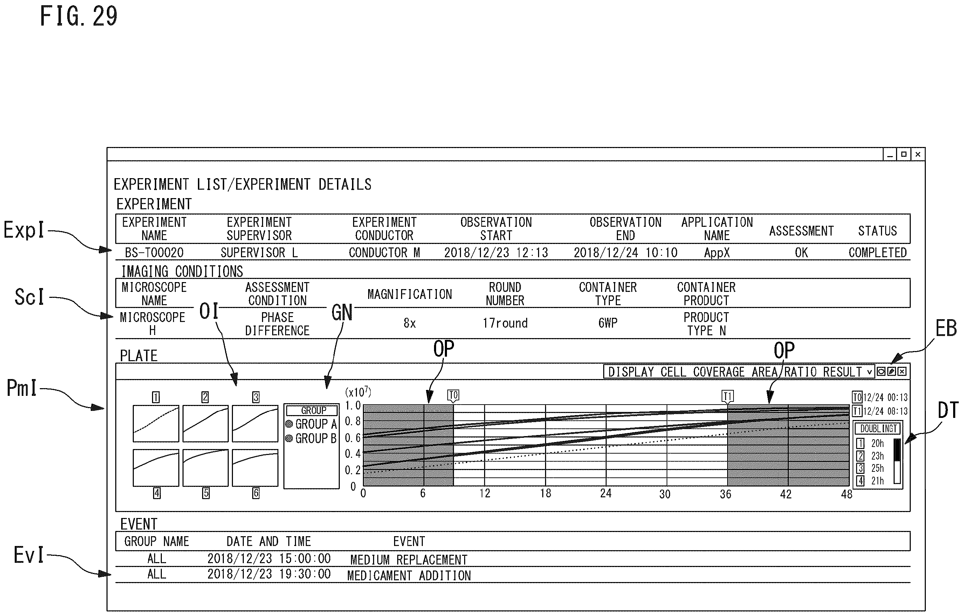

Fig. 28 is a diagram for describing an example of an operation for normalizing analysis results according to the third embodiment. Fig. 29 is a diagram for describing an example of an operation for denormalizing analysis results according to the third embodiment. As shown in Fig. 28, in a situation where an analysis result is displayed in a manner such that the number of target objects is plotted, the user performs, for example, a first operation such as a left-click at any position in a region Op outside the section set by the section-setting contents TA, TB. As a result, the display controller 133b executes normalization of the displayed analysis result. As shown in Fig. 29, in a situation where a normalized analysis result is displayed, the user performs, for example, a second operation such as a right-click at any position in the region Op outside the section set by the section-setting contents TA, TB. As a result, the display controller 133b denormalizes the displayed analysis result (normalized analysis result). That is to say, as a result of the first operation and the second operation having been performed positions in the region Op, the display controller 133b switches the analysis result shown in Fig. 28 and the analysis result shown in Fig. 29 to be displayed on the display 122.

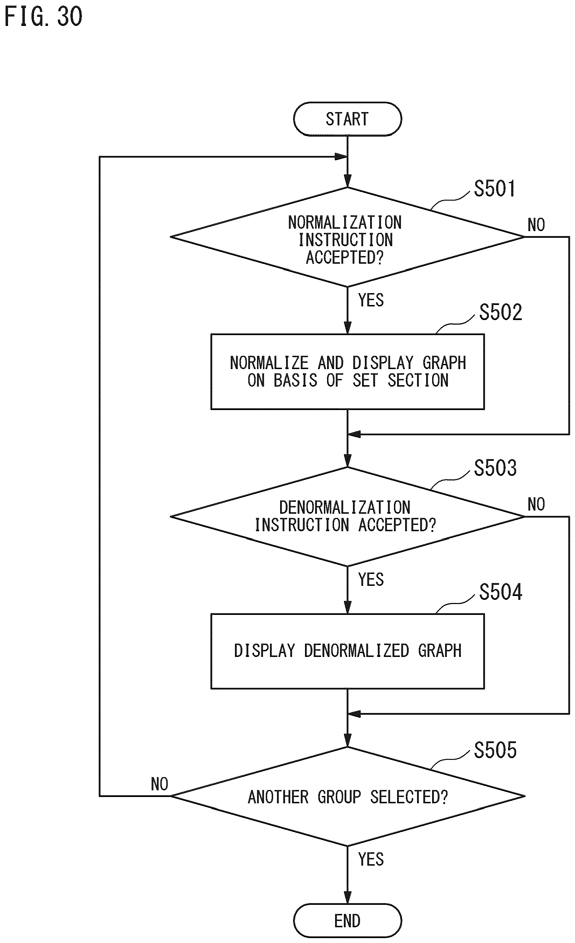

Fig. 30 is a flowchart showing an example of a process flow in the information processing device according to the third embodiment. In Step S501, the display controller 133b determines whether a normalization instruction has been accepted. For example, the display controller 133b determines whether it has accepted the execution of the first operation upon a user operation at a position in the region Op outside the section set by the section-setting contents TA, TB. At this time, if a normalization instruction has been accepted (Step S501: Yes), then in Step S502, the display controller 133b normalizes the graph on the basis of the section that has been set and indicates it in a display image, and the display controller 133b displays the display image on the display 122. For example, in the case where the execution of the first operation at the position in the region OP has been accepted, the display controller 133b acquires computation results related to normalization from the memory storage device 110 and indicates the graph normalized on the basis of the calculation results in a display image, and the display controller 133b displays the display image on the display 122. The calculation results related to normalization may be executed by the calculator 134b. On the other hand, if a normalization instruction has not been accepted (Step S501: No), the display controller 133b executes the process of Step S503.

In Step S503, the display controller 133b determines whether a denormalization instruction has been accepted. For example, the display controller 133b determines whether it has accepted the execution of the second operation upon a user operation at a position in the region Op outside the section set by the section-setting contents TA, TB. At this time, if a denormalization instruction has been accepted (Step S503: Yes), then in Step S504, the display controller 133b indicates the denormalized graph in a display image, and displays the display image on the display 122. For example, in the case where the execution of the second operation at the position in the region OP has been accepted, the display controller 133b denormalizes the graph to indicate the graph of the pre-normalization state in a display image, and displays the display image on the display 122. On the other hand, if a denormalization instruction has not been accepted (Step S503: No), the display controller 133b executes the process of Step S505.

In Step S505, the display controller 133b determines whether another group has been selected. At this time, if another group has been selected (Step S505: Yes), the display controller 133b ends the process of normalizing the analysis result or denormalizing it. On the other hand, if another group has not been selected (Step S505: No), the display controller 133b executes the process of Step S501.

### [Fourth Embodiment]

Hereunder, a fourth embodiment will be described. In the present embodiment, the same reference signs are given to configurations similar to those in the embodiment described above, and the descriptions thereof may be omitted or simplified in some cases.

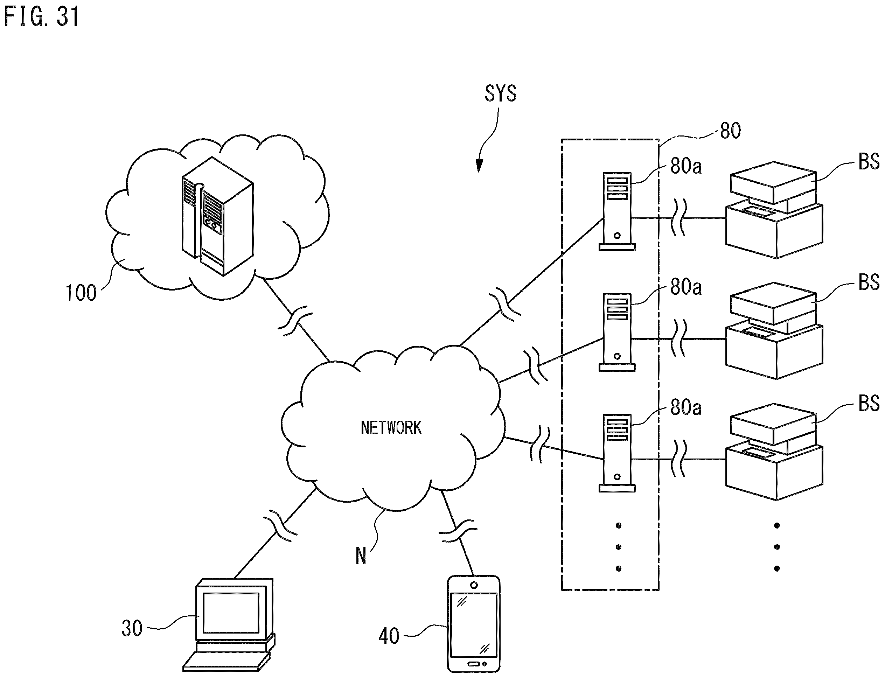

Fig. 31 is a diagram showing a configuration example of an information processing system according to the fourth embodiment. As shown in Fig. 31, an information processing system SYS has a first terminal 30, a second terminal 40, a terminal device 80, an information processing device 100, and culturing systems BS. The first terminal 30, the second terminal 40, the terminal device 80, and the information processing device 100 are connected to each other so as to be able to communicate with each other via a network N. The network N may be any of the Internet, a mobile communication network, and a local network, and may be a network in which networks of these several types are combined.

The terminal device 80 is composed of a plurality of gateway devices 80a. The gateway devices 80a are connected to the culturing systems BS in a wireless or wired manner. The information processing system SYS is configured such that a plurality of culturing systems BS are connected to the information processing device 100 via the terminal device 80, however, the invention is not limited to this example, and a single culturing system BS may be connected to the information processing device 100 via the terminal device 80. Furthermore, a plurality of information processing devices 100 or a single information processing device 100 may be provided in the information processing system SYS. Each information processing device 100 may include all of the various functions described in the above embodiments, or may include them in a distributed manner. That is to say, the information processing device 100 according to the present embodiment can be realized by cloud computing.

In the information processing system SYS, the user's terminal (such as first terminal 30 or second terminal 40) can connect to the information processing device 100, and observation results can be viewed or operated using a browser displayed on a display of the terminal. As a server, the information processing device 100 acquires, in an acquirer, observation results obtained from a time-lapse image of a target object. The information processing device 100, in an image generator, generates a display image including analysis results visually representing the observation results and an increase/decrease time indicating the amount of time required to increase or decrease a predetermined value related to the target object. When a setting for a section in the analysis results has been received, the information processing device 100, in the image generator, indicates in the display image the increase/decrease time corresponding to the section that has been set. Then, the information processing device 100, in an outputter, outputs the display image generated by the image generator to the user terminal via the network N.

In the embodiments described above, the information processing device 100 includes, for example, a computer system. The information processing device 100 reads an information processing program stored in a memory, and executes various processes in accordance with the read information processing program. Such an information processing program , for example, causes a computer to execute processes of: acquiring observation results obtained from a time-lapse image of a target object; indicating, in a display image, analysis results visually representing the observation results and an increase/decrease time indicating the amount of time required to increase or decrease a predetermined value related to the target object; and when a setting for a section in the analysis results has been received, indicating, in the display image, the increase/decrease time corresponding to the section that has been set. The information processing program may be recorded and provided on a computer-readable memory storage medium (for example, a non-transitory memory storage medium, or a non-transitory tangible medium). In the embodiments described above, when displaying visually represented analysis results in a graph, the calculator 134 of the information processing device 100 may calculate a single set of data to be displayed in the graph (for example, data of horizontal axis in the graph) through calculation performed on a plurality of observation images (for example, integrating or averaging).

The technical scope of the invention is not limited to the aspects described in the above embodiments and so forth. One or more of the requirements described in the above embodiments and so forth may be omitted. The requirements described in the above embodiments may be combined where appropriate. Furthermore, the contents of all documents cited in the detailed description of the present invention are incorporated herein by reference to the extent permitted by law.

### Description of Reference Signs

- 100:: Information processing device
- 110:: Memory storage device
- 121:: Inputter
- 122:: Display
- 131:: Communicator
- 132:: Acquirer
- 133:: Display controller
- 134:: Calculator
- 135:: Registerer

## Claims

1. An information processing device comprising:
an acquirer that acquires observation results obtained from a time-lapse image of a target object; and
a controller that indicates, in a display image, analysis results visually representing the observation results and an increase/decrease time indicating an amount of time required to increase or decrease a predetermined value related to the target object,
wherein, when a setting for a section in the analysis results has been received, the controller indicates, in the display image, the increase/decrease time corresponding to the section that has been set.

2. The information processing device according to claim 1,
wherein the controller receives a change of the section of time in the analysis results with respect to the display image and indicates, in the display image, the increase/decrease time corresponding to the section that has been received.

3. The information processing device according to claim 1 or 2,
wherein the target object includes cells, and
the increase/decrease time is a doubling time of the cells.

4. The information processing device according to any one of claims 1 to 3, comprising
a time calculator that calculates the increase/decrease time.

5. The information processing device according to any one of claims 1 to 3,
wherein the controller acquires the increase/decrease time from a memory storage device.

6. The information processing device according to any one of claims 1 to 5,
wherein the controller indicates, in the display image, a section-setting content for setting the section of time in the analysis results, and receives a setting of the section set by the section-setting content.

7. The information processing device according to claim 6,
wherein the controller indicates the section-setting content for setting the section in the display image, so as to be movable in the direction of a time axis in the analysis results.

8. The information processing device according to any one of claims 1 to 7,
wherein the controller indicates, in the display image, the analysis results normalized on the basis of the section.

9. The information processing device according to claim 8, comprising
a normalization calculator that executes a calculation related to normalization of the analysis results,
wherein the controller indicates, in the display image, the analysis results normalized on the basis of a calculation result of the normalization calculator.

10. The information processing device according to claim 8,
wherein the controller acquires from a memory storage device a normalization calculation result based on the section in the analysis results.

11. The information processing device according to any one of claims 8 to 10,
wherein the controller indicates, in the display image, the analysis results normalized according to a first operation, and indicates, in the display image, the analysis results denormalized according to a second operation different from the first operation.

12. The information processing device according to any one of claims 1 to 11,
wherein the controller indicates, in the display image, different representational effects inside and outside the section in the analysis results.

13. The information processing device according to any one of claims 1 to 12,
wherein the controller indicates, in the display image, information related to a date and a time of the section.

14. The information processing device according to any one of claims 1 to 13,
wherein the controller indicates, in the display image, the analysis results for the observation results selected by a user.

15. The information processing device according to claim 14,
wherein the controller indicates, in the display image, the analysis results for the observation results selected by a user from a list of the observation results.

16. The information processing device according to any one of claims 1 to 13,
wherein the controller indicates, in the display image, the plurality of analysis results for the plurality of observation results selected by a user in an overlapped manner.

17. The information processing device according to any one of claims 14 to 16,
wherein the controller indicates the display image in which the analysis results are switched according to a selection made by the user.

18. The information processing device according to any one of claims 1 to 17,
wherein the controller indicates, in the display image, the analysis results and the increase/decrease time so as to all fit in one screen.

19. The information processing device according to any one of claims 1 to 18,
wherein the controller indicates, in the display image, the analysis results in which information of the observation results are rendered in a graph.

20. The information processing device according to claim 19,
wherein the controller indicates, in the display image, a number of the target objects on an axis of the graph different from the time axis in the analysis results.

21. The information processing device according to claim 19,
wherein the controller indicates, in the display image, an area of the target object on an axis of the graph different from the time axis in the analysis results.

22. The information processing device according to claim 19,
wherein the controller indicates, in the display image, a perimeter length of the target object on an axis of the graph different from the time axis in the analysis results.

23. An information processing method comprising:
acquiring observation results obtained from a time-lapse image of a target object;
indicating, in a display image, analysis results visually representing the observation results and an increase/decrease time indicating an amount of time required to increase or decrease a predetermined value related to the target object; and
when a setting for a section in the analysis results has been received, indicating, in the display image, the increase/decrease time corresponding to the section that has been set.

24. An information processing program that causes a computer to execute processes of:
acquiring observation results obtained from a time-lapse image of a target object;
indicating, in a display image, analysis results visually representing the observation results and an increase/decrease time indicating an amount of time required to increase or decrease a predetermined value related to the target object; and
when a setting for a section in the analysis results has been received, indicating, in the display image, the increase/decrease time corresponding to the section that has been set.

25. An information processing system that outputs a display image to a user terminal by cloud computing, the information processing system comprising
a server,
wherein the server includes
an acquirer that acquires observation results obtained from a time-lapse image of a target object,
an image generator that generates the display image including analysis results visually representing the observation results and an increase/decrease time indicating an amount of time required to increase or decrease a predetermined value related to the target object, and
an outputter that outputs the display image generated by the image generator to the user terminal via a network,
wherein when a setting for a section in the analysis results has been received, the image generator indicates, in the display image, the increase/decrease time corresponding to the section that has been set.
